# EUROPÄISCHE PATENTSCHRIFT

(11) **EP 3 355 719 B1**
(45) Veröffentlichungstag und Bekanntmachung des Hinweises auf die Patenterteilung: **19.08.2020**
(21) Anmeldenummer: 15774592.8
(22) Anmeldetag: 30.09.2015
(51) Int. Cl.: A23L 27/00, C07C 235/48, C07C 235/60, A61K 8/42

(54) **VERWENDUNG BESTIMMTER HYDROXYBENZOESÄUREAMIDE ZUM MASKIEREN UNANGENEHMER GESCHMACKSEINDRÜCKE**
USE OF SOME HYDROXYBENZOIC ACID AMIDES FOR MASKING UNPLEASANT TASTE
UTILISATION DE CERTAINES AMIDES D'ACIDE HYDROXYBENZOIQUE POUR MASQUER UN GOUT DÉSAGRÉABLE

(43) Veröffentlichungstag der Anmeldung: 08.08.2018
(73) Patentinhaber: Symrise AG, 37603 Holzminden (DE)
(72) Erfinder: HANS, Joachim, 37603 Holzminden (DE); LEY, Jakob, 37603 Holzminden (DE); LANGER, Kathrin, 37586 Dassel (DE); PAETZ, Susanne, 37671 Höxter (DE)
(74) Vertreter: Eisenführ Speiser
(86) Internationale Anmeldenummer: PCT/EP2015/072620
(87) Internationale Veröffentlichungsnummer: WO 2017/054866

(56) Entgegenhaltungen:
- EP-A1- 2 386 211
- DE-A1-102004 041 496
- US-A1- 2011 158 919

### SEQUENCE LISTING

<110> Symrise AG
<120> Verwendung bestimmter Hydroxybenzoesäureamide zum Maskieren unangenehmer Geschmackseindrücke
<130> SM 5839-01EP
<160> 84
<170> PatentIn version 3.5
<210> 1
<400> 1
   000
<210> 2
   <211> 11
   <212> PRT
   <213> artificial sequence
<220>
   <223> peptide sequence
<400> 2
   Asn Phe Asn Asn Gln Leu Asp Gln Thr Pro Arg
   1 5 10
<210> 3
   <211> 10
   <212> PRT
   <213> artificial sequence
<220>
   <223> peptide sequence
<400> 3
   Ala Gly Asn Pro Asp Ile Glu His Pro Glu
   1 5 10
<210> 4
   <211> 10
   <212> PRT
   <213> artificial sequence
<220>
   <223> peptide sequence
<400> 4
   Asn Ala Leu Glu Pro Asp His Arg Val Glu
   1 5 10
<210> 5
   <211> 8
   <212> PRT
   <213> artificial sequence
<220>
   <223> peptide sequence
<400> 5
   Gly Asn Pro Asp Ile Glu His Pro
   1 5
<210> 6
   <211> 8
   <212> PRT
   <213> artificial sequence
<220>
   <223> peptide sequence
<400> 6
   Ile Tyr Pro Gly Cys Pro Ser Thr
   1 5
<210> 7
   <211> 8
   <212> PRT
   <213> artificial sequence
<220>
   <223> peptide sequence
<400> 7
   Lys Leu His Glu Asn Ile Ala Arg
   1 5
<210> 8
   <211> 8
   <212> PRT
   <213> artificial sequence
<220>
   <223> peptide sequence
<400> 8
   Leu Ala Gly Asn Gln Glu Gln Glu
   1 5
<210> 9
   <211> 7
   <212> PRT
   <213> artificial sequence
<220>
   <223> peptide sequence
<400> 9
   Ala Leu Glu Pro Asp His Arg
   1 5
<210> 10
   <211> 7
   <212> PRT
   <213> artificial sequence
<220>
   <223> peptide sequence
<400> 10
   Glu Gln Gly Gly Glu Gln Gly
   1 5
<210> 11
   <211> 7
   <212> PRT
   <213> artificial sequence
<220>
   <223> peptide sequence
<400> 11
   Glu Gln Pro Gln Gln Asn Glu
   1 5
<210> 12
   <211> 7
   <212> PRT
   <213> artificial sequence
<220>
   <223> peptide sequence
<400> 12
   Ile Gly Thr Leu Ala Gly Ala
   1 5
<210> 13
   <211> 7
   <212> PRT
   <213> artificial sequence
<220>
   <223> peptide sequence
<400> 13
   Asn Ala Met Phe Val Pro His
   1 5
<210> 14
   <211> 6
   <212> PRT
   <213> artificial sequence
<220>
   <223> peptide sequence
<400> 14
   Gly Met Ile Tyr Pro Gly
   1 5
<210> 15
   <211> 6
   <212> PRT
   <213> artificial sequence
<220>
   <223> peptide sequence
<400> 15
   His Asn Ile Gly Gln Thr
   1 5
<210> 16
   <211> 6
   <212> PRT
   <213> artificial sequence
<220>
   <223> peptide sequence
<400> 16
   Ile Tyr Pro Gly Cys Pro
   1 5
<210> 17
   <211> 6
   <212> PRT
   <213> artificial sequence
<220>
   <223> peptide sequence
<400> 17
   Asn Ala Leu Lys Pro Asp
   1 5
<210> 18
   <211> 5
   <212> PRT
   <213> artificial sequence
<220>
   <223> peptide sequence
<400> 18
   Phe Ile Gln Gly Val
   1 5
<210> 19
   <211> 5
   <212> PRT
   <213> artificial sequence
<220>
   <223> peptide sequence
<400> 19
   Asn Ala Leu Pro Glu
   1 5
<210> 20
   <211> 5
   <212> PRT
   <213> artificial sequence
<220>
   <223> peptide sequence
<400> 20
   Asn Asn Glu Asp Thr
   1 5
<210> 21
   <211> 5
   <212> PRT
   <213> artificial sequence
<220>
   <223> peptide sequence
<400> 21
   Ser Ala Glu Phe Gly
   1 5
<210> 22
   <211> 5
   <212> PRT
   <213> artificial sequence
<220>
   <223> peptide sequence
<400> 22
   Ser Ile Ile Asp Thr
   1 5
<210> 23
   <211> 5
   <212> PRT
   <213> artificial sequence
<220>
   <223> peptide sequence
<400> 23
   Tyr Glu Gly Asn Ser
   1 5
<210> 24
   <211> 4
   <212> PRT
   <213> artificial sequence
<220>
   <223> peptide sequence
<400> 24
   Leu Leu Leu Leu
   1
<210> 25
   <211> 4
   <212> PRT
   <213> artificial sequence
<220>
   <223> peptide sequence
<400> 25
   Asn Leu Gln Gly
   1
<210> 26
   <211> 4
   <212> PRT
   <213> artificial sequence
<220>
   <223> peptide sequence
<400> 26
   Ser Asp Asn Phe
   1
<210> 27
   <211> 15
   <212> PRT
   <213> artificial sequence
<220>
   <223> peptide sequence
<400> 27
   Val Glu Glu Leu Lys Pro Thr Pro Glu Gly Asp Leu Glu Ile Leu
   1 5 10 15
<210> 28
   <211> 13
   <212> PRT
   <213> artificial sequence
<220>
   <223> peptide sequence
<400> 28
   Gln Leu Phe Gly Pro Asn Val Asn Pro Trp His Asn Pro
   1               5                   10
<210> 29
   <211> 13
   <212> PRT
   <213> artificial sequence
<220>
   <223> peptide sequence
<400> 29
   Gln Leu Phe Asn Pro Ser Thr Asn Pro Trp His Ser Pro
   1               5                   10
<210> 30
   <211> 11
   <212> PRT
   <213> artificial sequence
<220>
   <223> peptide sequence
<400> 30
   Gly Gly Arg Gly Pro Pro Phe Ile Val Gly Gly
   1               5                   10
<210> 31
   <211> 11
   <212> PRT
   <213> artificial sequence
<220>
   <223> peptide sequence
<400> 31
   Gln Leu Phe Asn Pro Ser Thr Asn Pro Trp His
   1               5                   10
<210> 32
   <211> 9
   <212> PRT
   <213> artificial sequence
<220>
   <223> peptide sequence
<400> 32
   Gly Gly Arg Gly Pro Pro Phe Ile Val
   1 5
<210> 33
   <211> 9
   <212> PRT
   <213> artificial sequence
<220>
   <223> peptide sequence
<400> 33
   Gln Leu Phe Asn Pro Ser Thr Asn Pro
   1 5
<210> 34
   <211> 9
   <212> PRT
   <213> artificial sequence
<220>
   <223> peptide sequence
<400> 34
   Arg Gly Pro Pro Phe Ile Val Gly Gly
   1 5
<210> 35
   <211> 9
   <212> PRT
   <213> artificial sequence
<220>
   <223> peptide sequence
<400> 35
   Arg Gly Pro Pro Gly Gly Gly Phe Phe
   1 5
<210> 36
   <211> 8
   <212> PRT
   <213> artificial sequence
<220>
   <223> peptide sequence
<400> 36
   Gly Gly Arg Pro Phe Phe Gly Gly
   1 5
<210> 37
   <211> 6
   <212> PRT
   <213> artificial sequence
<220>
   <223> peptide sequence
<400> 37
   Gln Leu Phe Asn Pro Ser
   1               5
<210> 38
   <211> 27
   <212> PRT
   <213> artificial sequence
<220>
   <223> peptide sequence
<400> 38
   Ala Gln Thr Gln Ser Leu Val Tyr Pro Phe Pro Gly Pro Ile Pro Asn
   1               5                   10                  15
   Ser Leu Pro Gln Asn Ile Pro Pro Leu Thr Gln
               20                  25
<210> 39
   <211> 17
   <212> PRT
   <213> artificial sequence
<220>
   <223> peptide sequence
<400> 39
   Tyr Gln Gln Pro Val Leu Gly Pro Val Arg Gly Pro Phe Pro Ile Ile
   1               5                   10                  15
   Val
<210> 40
   <211> 14
   <212> PRT
   <213> artificial sequence
<220>
   <223> peptide sequence
<400> 40
   Pro Val Leu Gly Pro Val Arg Gly Pro Phe Pro Ile Ile Val
   1               5                   10
<210> 41
   <211> 13
   <212> PRT
   <213> artificial sequence
<220>
   <223> peptide sequence
<400> 41
   Ser Leu Val Tyr Pro Phe Pro Gly Pro Ile His Asn Ser
   1               5                   10
<210> 42
   <211> 13
   <212> PRT
   <213> artificial sequence
<220>
   <223> peptide sequence
<400> 42
   Val Pro Leu Gly Thr Gln Tyr Thr Asp Ala Pro Ser Phe
   1               5                   10
<210> 43
   <211> 12
   <212> PRT
   <213> artificial sequence
<220>
   <223> peptide sequence
<400> 43
   Phe Phe Val Ala Pro Phe Pro Glu Val Phe Gly Lys
   1               5                   10
<210> 44
   <211> 12
   <212> PRT
   <213> artificial sequence
<220>
   <223> peptide sequence
<400> 44
   Phe Phe Val Ala Pro Phe Pro Gln Val Phe Gly Lys
   1               5                   10
<210> 45
   <211> 11
   <212> PRT
   <213> artificial sequence
<220>
   <223> peptide sequence
<400> 45
   Leu Val Tyr Pro Phe Pro Gly Pro Ile His Asn
   1 5 10
<210> 46
   <211> 10
   <212> PRT
   <213> artificial sequence
<220>
   <223> peptide sequence
<400> 46
   Pro Val Arg Gly Pro Phe Pro Ile Ile Val
   1 5 10
<210> 47
   <211> 10
   <212> PRT
   <213> artificial sequence
<220>
   <223> peptide sequence
<400> 47
   Val Tyr Pro Phe Pro Gly Pro Ile Pro Asn
   1 5 10
<210> 48
   <211> 10
   <212> PRT
   <213> artificial sequence
<220>
   <223> peptide sequence
<400> 48
   Val Tyr Pro Phe Pro Pro Ile Gly Asn His
   1 5 10
<210> 49
   <211> 10
   <212> PRT
   <213> artificial sequence
<220>
   <223> peptide sequence
<400> 49
   Tyr Leu Gly Tyr Leu Glu Gln Leu Leu Arg
   1 5 10
<210> 50
   <211> 10
   <212> PRT
   <213> artificial sequence
<220>
   <223> peptide sequence
<400> 50
   Tyr Pro Phe Pro Gly Pro Ile His Asn Ser
   1               5                   10
<210> 51
   <211> 10
   <212> PRT
   <213> artificial sequence
<220>
   <223> peptide sequence
<400> 51
   Tyr Pro Phe Pro Gly Pro Ile Pro Asn Ser
   1               5                   10
<210> 52
   <211> 9
   <212> PRT
   <213> artificial sequence
<220>
   <223> peptide sequence
<400> 52
   Pro Phe Pro Gly Pro Ile Pro Asn Ser
   1               5
<210> 53
   <211> 9
   <212> PRT
   <213> artificial sequence
<220>
   <223> peptide sequence
<400> 53
   Tyr Pro Phe Pro Gly Pro His Ile Asn
   1               5
<210> 54
   <211> 9
   <212> PRT
   <213> artificial sequence
<220>
   <223> peptide sequence
<400> 54
   Tyr Pro Phe Pro Gly Pro Ile Pro Asn
   1 5
<210> 55
   <211> 8
   <212> PRT
   <213> artificial sequence
<220>
   <223> peptide sequence
<400> 55
   Phe Ala Leu Pro Gln Tyr Leu Lys
   1 5
<210> 56
   <211> 8
   <212> PRT
   <213> artificial sequence
<220>
   <223> peptide sequence
<400> 56
   Gly Pro Val Arg Gly Pro Phe Pro
   1 5
<210> 57
   <211> 8
   <212> PRT
   <213> artificial sequence
<220>
   <223> peptide sequence
<400> 57
   Leu Gly Tyr Leu Glu Gln Leu Leu
   1 5
<210> 58
   <211> 8
   <212> PRT
   <213> artificial sequence
<220>
   <223> peptide sequence
<400> 58
   Arg Gly Pro Phe Pro Ile Ile Val
   1 5
<210> 59
   <211> 8
   <212> PRT
   <213> artificial sequence
<220>
   <223> peptide sequence
<400> 59
   Arg Gly Pro Gly Pro Ile Ile Val
   1               5
<210> 60
   <211> 7
   <212> PRT
   <213> artificial sequence
<220>
   <223> peptide sequence
<400> 60
   Ala Pro Phe Pro Glu Val Phe
   1               5
<210> 61
   <211> 7
   <212> PRT
   <213> artificial sequence
<220>
   <223> peptide sequence
<400> 61
   Ala Tyr Phe Tyr Pro Glu Leu
   1               5
<210> 62
   <211> 7
   <212> PRT
   <213> artificial sequence
<220>
   <223> peptide sequence
<400> 62
   Phe Tyr Pro Glu Leu Phe Arg
   1               5
<210> 63
   <211> 7
   <212> PRT
   <213> artificial sequence
<220>
   <223> peptide sequence
<400> 63
   Pro Phe Pro Gly Pro Ile Pro
   1 5
<210> 64
   <211> 7
   <212> PRT
   <213> artificial sequence
<220>
   <223> peptide sequence
<400> 64
   Arg Gly Pro Phe Pro Ile Val
   1 5
<210> 65
   <211> 7
   <212> PRT
   <213> artificial sequence
<220>
   <223> peptide sequence
<400> 65
   Tyr Pro Phe Pro Gly Pro Ile
   1 5
<210> 66
   <211> 6
   <212> PRT
   <213> artificial sequence
<220>
   <223> peptide sequence
<400> 66
   Pro Val Leu Gly Pro Val
   1 5
<210> 67
   <211> 6
   <212> PRT
   <213> artificial sequence
<220>
   <223> peptide sequence
<400> 67
   Val Arg Gly Pro Phe Pro
   1 5
<210> 68
   <211> 6
   <212> PRT
   <213> artificial sequence
<220>
   <223> peptide sequence
<400> 68
   Phe Tyr Pro Glu Leu Phe
   1 5
<210> 69
   <211> 6
   <212> PRT
   <213> artificial sequence
<220>
   <223> peptide sequence
<400> 69
   Gly Pro Phe Pro Ile Val
   1 5
<210> 70
   <211> 6
   <212> PRT
   <213> artificial sequence
<220>
   <223> peptide sequence
<400> 70
   Pro Phe Pro Gly Pro Ile
   1 5
<210> 71
   <211> 6
   <212> PRT
   <213> artificial sequence
<220>
   <223> peptide sequence
<400> 71
   Pro Phe Pro Ile Ile Val
   1 5
<210> 72
   <211> 5
   <212> PRT
   <213> artificial sequence
<220>
   <223> peptide sequence
<400> 72
   Asp Ile Lys Gln Met
   1 5
<210> 73
   <211> 5
   <212> PRT
   <213> artificial sequence
<220>
   <223> peptide sequence
<400> 73
   Glu Ile Val Pro Asn
   1 5
<210> 74
   <211> 5
   <212> PRT
   <213> artificial sequence
<220>
   <223> peptide sequence
<400> 74
   Asn Glu Asn Leu Leu
   1 5
<210> 75
   <211> 5
   <212> PRT
   <213> artificial sequence
<220>
   <223> peptide sequence
<400> 75
   Pro Gly Pro Ile Pro
   1 5
<210> 76
   <211> 4
   <212> PRT
   <213> artificial sequence
<220>
   <223> peptide sequence
<400> 76
   Glu Val Leu Asn
   1
<210> 77
   <211> 4
   <212> PRT
   <213> artificial sequence
<220>
   <223> peptide sequence
<400> 77
   Leu Pro Gln Glu
   1
<210> 78
   <211> 4
   <212> PRT
   <213> artificial sequence
<220>
   <223> peptide sequence
<400> 78
   Val Tyr Pro Phe
   1
<210> 79
   <211> 6
   <212> PRT
   <213> artificial sequence
<220>
   <223> peptide sequence
<400> 79
   Gly Pro Phe Pro Val Ile
   1 5
<210> 80
   <211> 8
   <212> PRT
   <213> artificial sequence
<220>
   <223> peptide sequence
<400> 80
   Phe Ala Leu Pro Glu Tyr Leu Lys
   1 5
<210> 81
   <211> 7
   <212> PRT
   <213> artificial sequence
<220>
   <223> peptide sequence
<400> 81
   Arg Gly Pro Pro Phe Ile Val
   1 5
<210> 82
   <211> 4
   <212> PRT
   <213> artificial sequence
<220>
   <223> peptide sequence
<400> 82
   Tyr Pro Phe Pro
   1
<210> 83
   <211> 5
   <212> PRT
   <213> artificial sequence
<220>
   <223> peptide sequence
<400> 83
   Ile Pro Ala Val Phe
   1 5
<210> 84
   <211> 4
   <212> PRT
   <213> artificial sequence
<220>
   <223> peptide sequence
<400> 84
   Tyr Gly Leu Phe
   1

## Beschreibung

Die vorliegende Erfindung betrifft primär die Verwendung bestimmter Hydroxybenzoesäureamide, deren Salzen und deren Gemischen zum Maskieren eines unangenehmen Geschmackseindrucks, vorzugsweise eines bitteren, adstringierenden, pappigen, staubigen, trockenen, mehligen, ranzigen und/oder metallischen Geschmacks oder Nachgeschmacks, eines oder mehrerer bestimmter unangenehm schmeckender Peptide oder bestimmter Aminosäuremischungen. Ferner betrifft die Erfindung entsprechende Verfahren zur Maskierung unangenehmer Geschmackseindrücke und neue Zubereitungen.

Weitere Aspekte der vorliegenden Erfindung ergeben sich aus der nachfolgenden Beschreibung, dem Beispielteil sowie insbesondere den beigefügten Patentansprüchen.

Nahrungs- oder Genussmittel enthalten häufig verschiedene Bitterstoffe, die zwar einerseits in Maßen erwünscht und charakteristisch sind (z.B. Coffein in Tee oder Kaffee, Chinin in sogenannten Bitter-Lemon-Getränken oder Hopfenextrakte in Bier), andererseits den Wert aber auch stark mindern können (z.B. Flavonoidglycoside und Limonoide in Zitrus-Säften; bitterer Nachgeschmack vieler künstlicher Süßstoffe wie Aspartam oder Saccharin; hydrophobe Aminosäuren und/oder Peptide in Käse). (Bittere) Peptide finden sich häufig als Mischungen in Proteinhydrolysaten, speziell Hydrolysaten von Milchproteinen oder Fraktionen davon (wie der Casein- oder der

Molkenfraktion), welche ebenfalls regelmäßig Nahrungsmitteln zugesetzt werden (Sportlernahrung, etc.). Ebenso sind Peptide, die bei der Käsereifung entstehen können, oft als bitter beschrieben. Auch Hydrolysate von Pflanzenproteinfraktionen (Soja, Alfalfa, Erbse, Bohne, Lupine, etc.) zeichnen sich oft durch einen bitteren Geschmack aus.

Um die wertgebenden und notwendigen Inhaltsstoffe (z.B. Aminosäuren oder Peptide) in den Nahrungsmitteln belassen zu können, ist es wünschenswert, Stoffe zu finden, die unangenehme Geschmackseindrücke, insbesondere bittere, adstringierende und/oder metallische Geschmackseindrücke wirkungsvoll unterdrücken oder zumindest vermindern können.

Zwar kennt man einige Stoffe, die den bitteren Geschmack (partiell) unterdrücken können, doch zeigen viele in der Anwendung starke Limitationen.

Kennison et al. (US 2011 0158919) beschreiben eine Aromazusammensetzung mit Süßstoffen zur Maskierung bzw. Unterdrückung von Fehlnoten in Lebensmitteln.

Ley et al. (DE 10 2004 041 496) beschreiben die Verwendung von Hydroxybenzoesäureamiden und deren Verwendung zur Maskierung von bitterem Geschmack, aber nicht zur Maskierung von speziellen Peptiden.

Ley et al. (EP 2 386 211) beschreiben auch die Verwendung von Rubusosid zum Verringern beziehungsweise Unterdrücken von bestimmten Fehlnoten wie bitter, sauer oder adstringierend, sowie orale Zubereitungen, welche einen Stoff mit Fehlnote sowie Rubusosid enthalten.

Dong et al. (CN 101147800) beschreiben die Maskierung des bitteren Geschmacks von Casein-Phosphopeptiden durch Komplexierung mit Cyclodextranen, jedoch ist diese Methode hochspezifisch für die eingesetzten Phosphopeptide.

Tanisawa et al. (JP 2009 278,917) beschreiben die Verwendung eines essbaren Koagulationsmittels zur Entbitterung von Lebensmitteln, wodurch allerdings die Bioverfügbarkeit der bitteren Stoffe möglicherweise negativ beeinflusst wird.

Urata et al. (JP 2011 162,539) beschreiben die Verwendung eines stilben-haltigen Pflanzenextrakts in Verbindung mit verzweigtkettigen Cyclodextrinen zur Maskierung des Bittergeschmacks von Milchpeptiden.

Hamaguchi et al. (JP 2012 110,248) beschreiben die Verwendung von Polyaminosäuren mit einem Molekulargewicht von mind. 500 Da zur Maskierung des Bittergeschmacks von Getränken und Lebensmitteln.

Es besteht jedoch ständiger Bedarf, Stoffe zu finden, die zur Maskierung unangenehmer Geschmackseindrucke bestimmter Substanzen geeignet sind, insbesondere solche Stoffe, die leicht zugänglich sind. Vorzugsweise sollen dabei solche Stoffe angegeben werden, die auch in sehr geringen Konzentrationen den unangenehmen Geschmackseindruck reduzieren oder sogar vollständig unterdrücken können, wobei es besonders vorteilhaft ist, wenn die Stoffe bei einer solchen Konzentration nahezu keinen Eigengeschmack besitzen und damit weitere, in der Regel nicht unangenehme Geschmacksqualitäten nicht beeinflussen.

Die vorliegende Erfindung basiert auf der Aufgabe, eben solche Stoffe zu finden.

Die gestellte Aufgabe wird erfindungsgemäß gelöst durch Verwendung eines, zweier oder mehrerer verschiedener Hydroxybenzoesäureamide der Formel (I) 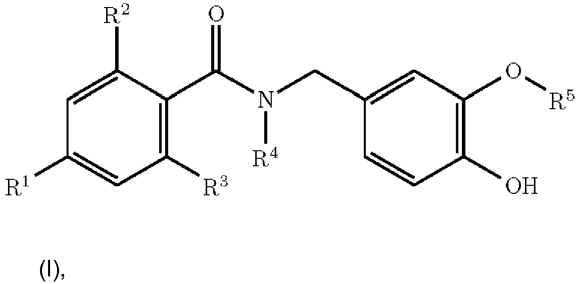 wobei
R¹ und R² unabhängig voneinander Wasserstoff, Hydroxy oder Methoxy bedeuten, vorzugsweise Wasserstoff oder Hydroxy, mit der Maßgabe, dass mindestens einer der Reste R¹ oder R² Hydroxy bedeutet,
   und
entweder
   R³ Wasserstoff oder Hydroxy, vorzugsweise Wasserstoff,
   und
   R⁴ Wasserstoff bedeutet
oder
   R³ und R⁴ gemeinsam eine Gruppe -O-C(=O)- darstellen und damit einen Ring in Form eines cyclischen Carbamats bilden,
   und
   R⁵ Wasserstoff oder Methyl, vorzugsweise Methyl, bedeutet,
oder
eines, zweier oder mehrerer verschiedener Salze eines, zweier oder mehrerer verschiedener Hydroxybenzoesäureamide der Formel (I) wie oben definiert oder
eines Gemisches eines, zweier oder mehrerer verschiedener Hydroxybenzoesäureamide der Formel (I) wie oben definiert mit einem, zwei oder mehreren verschiedenen Salzen eines, zweier oder mehrerer verschiedener Hydroxybenzoesäureamide der Formel (I) wie oben definiert
zum Maskieren eines unangenehmen Geschmackseindrucks, vorzugsweise eines bitteren, adstringierenden, pappigen, staubigen, trockenen, mehligen, ranzigen und/oder metallischen Geschmacks oder Nachgeschmacks, eines oder mehrerer unangenehm schmeckender Stoffe, wobei der bzw. einer, mehrere oder sämtliche der unangenehmen Stoffe ausgewählt ist bzw. sind aus der Gruppe bestehend aus Aminosäuremischungen enthaltend oder bestehend aus, jeweils bezogen auf das Gewicht, 40 bis 50 Teilen L-Leucin, 20 bis 30 Teilen L-Isoleucin und 20 bis 40 Teilen L-Valin, oder 10 bis 30 Teilen L-Isoleucin, 25 bis 45 Teilen L-Leucin, 5 bis 15 Teilen L-Phenylalanin und 5 bis 15 Teilen L-Tryptophan, und Peptiden mit den Sequenzen GKHQQEEENEGG (SEQ ID NO: 1), NFNNQLDQTPR (SEQ ID NO: 2), AGNPDIEHPE (SEQ ID NO: 3), NALEPDHRVE (SEQ ID NO: 4), GNPDIEHP (SEQ ID NO: 5), IYPGCPST (SEQ ID NO: 6), KLHENIAR (SEQ ID NO: 7), LAGNQEQE (SEQ ID NO: 8), ALEPDHR (SEQ ID NO: 9), EQGGEQG (SEQ ID NO: 10), EQPQQNE (SEQ ID NO: 11), IGTLAGA (SEQ ID NO: 12), NAMFVPH (SEQ ID NO: 13), GMIYPG (SEQ ID NO: 14), HNIGQT (SEQ ID NO: 15), IYPGCP (SEQ ID NO: 16), NALKPD (SEQ ID NO: 17), FIQGV (SEQ ID NO: 18), NALPE (SEQ ID NO: 19), NNEDT (SEQ ID NO: 20), SAEFG (SEQ ID NO: 21), SIIDT (SEQ ID NO: 22), YEGNS (SEQ ID NO: 23), LLLL (SEQ ID NO: 24), NLQG (SEQ ID NO: 25), SDNF (SEQ ID NO: 26), EGG, GAL, GGL, KPF, LLL, PPG, AD, EG, EY, GE, GF, GI, IA, IP, IS, KP, LK, PR, PY, RG, RP, RR, VF, VI, W, VY, VEELKPTPEGDLEIL (SEQ ID NO: 27), LKP, LVL, DL, ID, LD, LE, LV, WE, QLFGPNVNPWHNP (SEQ ID NO: 28), QLFNPSTNPWHSP (SEQ ID NO: 29), GGRGPPFIVGG (SEQ ID NO: 30), QLFNPSTNPWH (SEQ ID NO: 31), GGRGPPFIV (SEQ ID NO: 32), QLFNPSTNP (SEQ ID NO: 33), RGPPFIVGG (SEQ ID NO: 34), RGPPGGGFF (SEQ ID NO: 35), GGRPFFGG (SEQ ID NO: 36), QLFNPS (SEQ ID NO: 37), GFG, GLL, GW, LQL, RPG, EF, FG, FV, GL, GR, GV, IE, II, IL, IQ, LF, LI, RF, VA, WF, YF, YG, AQTQSLVYPFPGPIPNSLPQNIPPLTQ (SEQ ID NO: 38), YQQPVLGPVRGPFPIIV (SEQ ID NO: 39), PVLGPVRGPFPIIV (SEQ ID NO: 40), SLVYPFPGPIHNS (SEQ ID NO: 41), VPLGTQYTDAPSF (SEQ ID NO: 42), FFVAPFPEVFGK (SEQ ID NO: 43), FFVAPFPQVFGK (SEQ ID NO: 44), LVYPFPGPIHN (SEQ ID NO: 45), PVRGPFPIIV (SEQ ID NO: 46), VYPFPGPIPN (SEQ ID NO: 47), VYPFPPIGNH (SEQ ID NO: 48), YLGYLEQLLR (SEQ ID NO: 49), YPFPGPIHNS (SEQ ID NO: 50), YPFPGPIPNS (SEQ ID NO: 51), PFPGPIPNS (SEQ ID NO: 52), YPFPGPHIN (SEQ ID NO: 53), YPFPGPIPN (SEQ ID NO: 54), FALPQYLK (SEQ ID NO: 55), GPVRGPFP (SEQ ID NO: 56), LGYLEQLL (SEQ ID NO: 57), RGPFPIIV (SEQ ID NO: 58), RGPGPIIV (SEQ ID NO: 59), APFPEVF (SEQ ID NO: 60), AYFYPEL (SEQ ID NO: 61), FYPELFR (SEQ ID NO: 62), PFPGPIP (SEQ ID NO: 63), RGPFPIV (SEQ ID NO: 64), YPFPGPI (SEQ ID NO: 65), PVLGPV (SEQ ID NO: 66), VRGPFP (SEQ ID NO: 67), FYPELF (SEQ ID NO: 68), GPFPIV (SEQ ID NO: 69), PFPGPI (SEQ ID NO: 70), PFPIIV (SEQ ID NO: 71), DIKQM (SEQ ID NO: 72), EIVPN (SEQ ID NO: 73), NENLL (SEQ ID NO: 74), PGPIP (SEQ ID NO: 75), EVLN (SEQ ID NO: 76), LPQE (SEQ ID NO: 77), VYPF (SEQ ID NO: 78), APK, EEN, FLL, FPK, FPP, LRF, LRL, PFP, PGP, PPF, RGP, VW, VYP, YPF, AF, AL, AV, DA, EI, EL, FF, Fl, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI (SEQ ID NO: 79), FALPEYLK (SEQ ID NO: 80), VG, RGPPFIV (SEQ ID NO: 81), GRP, FFF, WW, WWW, YPFP (SEQ ID NO: 82), FPF, IPAVF (SEQ ID NO: 83), LLF oder YGLF (SEQ ID NO: 84).

Entsprechend der dem Fachmann geläufigen Bedeutung des Einbuchstabencodes für Aminosäuresequenzen (wie vorstehend zur Angabe der erfindungsgemäß zu maskierenden bitteren Peptide verwendet) gelten folgende Bedeutungen:

| | |
|---|---|
| Alanin | A |
| Arginin | R |
| Asparagin | N |
| Asparaginsäure | D |
| Cystein | C |
| Glutamin | Q |
| Glutaminsäure | E |
| Glycin | G |
| Histidin | H |
| Isoleucin | I |
| Leucin | L |
| Lysin | K |
| Methionin | M |
| Phenylalanin | F |
| Prolin | P |
| Serin | S |
| Threonin | T |
| Tryptophan | W |
| Tyrosin | Y |
| Valin | V |

Unter Maskieren wird im Rahmen des vorliegenden Textes eine Reduzierung, d.h. eine Verminderung, oder eine vollständige Unterdrückung verstanden. Das Maskieren des unangenehmen Geschmackseindrucks bedeutet damit im Ergebnis regelmäßig eine Geschmacksverbesserung, insbesondere in Bezug auf bittere, adstringierende, pappige, staubige, trockene, mehlige, ranzige und/oder metallische Geschmackseindrücke.

Bei den oben genannten Peptiden wird gemäß der üblichen Schreibweise das N-terminale Ende des Peptids links aufgeführt. Des weiteren werden die üblichen Abkürzungen für die Aminosäuren verwendet.

Besonders bevorzugt und erfindungsgemäß besonders geeignet sind die Verbindungen
2,4-Dihydroxy-N-[(4-hydroxy-3-methoxy-phenyl)methyl]benzamid (Verbindung 1),
5,7-Dihydroxy-3-[(4-hydroxy-3-methoxy-phenyl)methyl]-1,3-benzoxazin-2,4-dion (Verbindung 2),
4-Hydroxy-N-[(4-hydroxy-3-methoxy-phenyl)methyl]benzamide (Verbindung 3) und 2-Hydroxy-N-[(4-hydroxy-3-methoxy-phenyl)methyl]benzamide (Verbindung 4)
und deren Salze.

Zur Verdeutlichung sind im folgenden die Strukturen der Verbindungen 1 bis 4 aufgeführt:

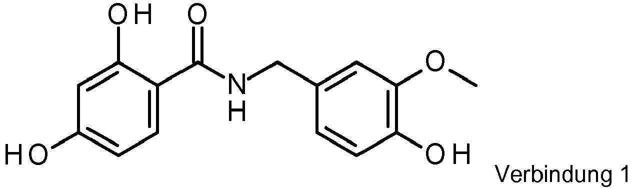

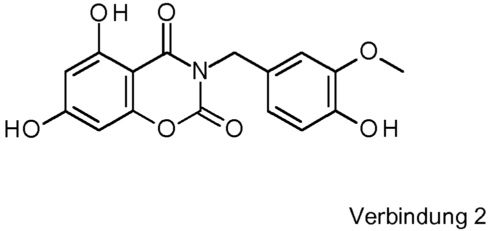

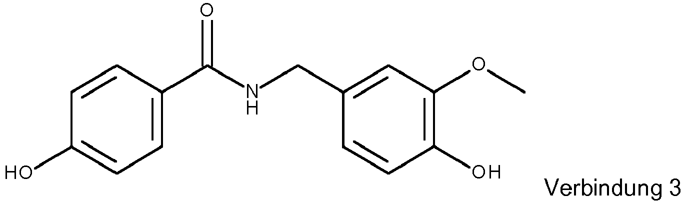

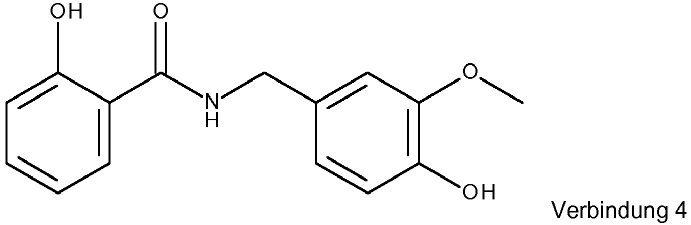

Unangenehm schmeckende Peptide bzw. Aminosäuremischungen wie hierin beschrieben sind solche, die
(a) bitter, adstringierend, pappig, staubig, trocken, mehlig, ranzig und/oder metallisch schmecken und/oder
(b) einen bitteren, adstringierenden, pappigen, staubigen, trockenen, mehligen, ranzigen oder metallischen Nachgeschmack haben.

Die unangenehm schmeckenden Peptide bzw. Aminosäuremischungen können noch weitere, in der Regel nicht unangenehme Geschmacks- und/oder Geruchsqualitäten besitzen. Als weitere, im Sinne der vorliegenden Erfindung nicht unangenehme Geschmacksqualitäten sind z.B. die Eindrücke würzig, umami, süß, salzig, sauer, scharf, kühlend, wärmend, brennend oder kribbelnd zu nennen.

Es war besonders überraschend, dass sich die erfindungsgemäß zu verwendenden Verbindungen der Formel (I) und deren Salze vorteilhafterweise dazu eignen, unangenehme Geschmackseindrücke der hierin beschriebenen Peptide bzw. Aminosäuremischungen zu maskieren. Im Beispielteil weiter unten sind Vergleichsdaten zu weiteren - nicht in der Liste gemäß Anspruch 1 enthaltenen - bitteren Peptiden, dessen Bittergeschmack nicht reduziert wird, und Vergleichsdaten zu einer nicht unter die hierin beschriebene Formel (I) fallenden Verbindung gezeigt (diese belegen, dass strukturell ähnliche, nicht erfindungsgemäß zu verwendende Verbindungen im Gegensatz zu den erfindungsgemäß zu verwendenden Verbindungen nicht - oder nicht so gut - geeignet sind, den Bittergeschmack der Peptide der Liste gemäß Anspruch 1 zu maskieren).

In Salzen eines erfindungsgemäß zu verwendenden Hydroxybenzoesäureamids der obigen Formel (I) sind eine, mehrere oder sämtliche Hydroxy-Gruppen des Hydroxybenzoesäureamids deprotoniert. Es liegt dann eine entsprechende Menge von Gegenkationen vor, wobei diese vorzugsweise ausgewählt sind aus der Gruppe bestehend aus Na+ und K+.

Selbstverständlich können die verschiedenen erfindungsgemäß zu verwendenden Hydroxybenzoesäureamide und deren Salze jeweils alleine oder als Gemische erfindungsgemäß verwendet werden.

Überraschenderweise wurde gefunden, dass die erfindungsgemäß zu verwendenden Hydroxybenzoesäureamide auch in sehr geringen Konzentrationen den unangenehmen Geschmackseindruck, insbesondere den bitteren Geschmackseindruck reduzieren oder sogar vollständig unterdrücken können, wobei es besonders vorteilhaft ist, dass die erfindungsgemäß zu verwendenden Hydroxybenzoesäureamide und deren Salze bei einer solchen Konzentration nahezu keinen Eigengeschmack besitzen und die weiteren, in der Regel nicht unangenehmen Geschmacksqualitäten nicht beeinflussen. Dementsprechend ist es im Rahmen der vorliegenden Erfindung gemäß einem bevorzugten Aspekt wünschenswert, dass die Konzentration der erfindungsgemäß zu verwendenden Hydroxybenzoesäureamide und deren Salzen in einem Bereich liegt, in dem die Hydroxybenzoesäureamide bzw. deren Salze (nahezu) keinen Eigengeschmack besitzen.

Vorzugsweise werden die hierin beschriebenen Hydroxybenzoesäureamide und deren Salze in Kombination mit einer, zwei oder mehreren weiteren Substanzen zum Maskieren eines unangenehmen, insbesondere bitteren, Geschmackseindrucks eines oder mehrerer unangenehm schmeckender Stoffe verwendet, vorzugsweise solchen wie hierin weiter unten beschrieben.

Besonders bevorzugt werden die hierin beschrieben Hydroxybenzoesäureamide und deren Salze in einer Zubereitung ausgewählt aus der Gruppe bestehend aus
- der Ernährung oder der Mundpflege dienenden, oralen pharmazeutischen oder kosmetischen, zur Applikation im Bereich des Kopfes dienenden Zubereitungen (Fertigwaren), und
- Halbfertigwaren, insbesondere solchen zur Herstellung einer der Ernährung oder der Mundpflege dienenden, oralen pharmazeutischen oder kosmetischen, zur Applikation im Bereich des Kopfes dienenden Zubereitung (Fertigwaren), vorzugsweise in Form einer Riech-, Aroma- oder Geschmackstoffkomposition oder Würzmischung,
eingesetzt (für bevorzugte Mengen an Hydroxybenzoesäureamide und deren Salzen siehe weiter unten).

Ein weiterer Aspekt der vorliegenden Erfindung betrifft derartige Zubereitungen. Eine solche Zubereitung ist ausgewählt aus der Gruppe bestehend aus
(A) der Ernährung, dem Genuss oder der Mundpflege dienenden, oralen pharmazeutischen oder kosmetischen, zur Applikation im Bereich des Kopfes dienenden Zubereitungen (Fertigwaren), und
(B) Halbfertigwaren, insbesondere solchen zur Herstellung einer der Ernährung, dem Genuss oder der Mundpflege dienenden, oralen pharmazeutischen oder kosmetischen, zur Applikation im Bereich des Kopfes dienenden Zubereitung (Fertigwaren), vorzugsweise in Form einer Riech-, Aroma- oder Geschmackstoffkomposition oder Würzmischung,
   enthaltend
   (i) eine, zwei oder mehrere verschiedene Hydroxybenzoesäureamide der Formel (I), wie oben definiert, oder ein, zwei oder mehrere verschiedene Salze eines, zweier oder mehrerer verschiedener Hydroxybenzoesäureamide der Formel (I), wie oben definiert, oder ein Gemisches eines, zweier oder mehrerer verschiedener Hydroxybenzoesäureamide der Formel (I), wie oben definiert, mit einem, zwei oder mehreren verschiedenen Salzen eines, zweier oder mehrerer verschiedener Hydroxybenzoesäureamide der Formel (I), wie oben definiert,
      in einer Menge von
      (A) 0,0001 bis 0,5 Gew.-%, vorzugsweise 0,001 bis 0,1 Gew.-%, bezogen auf das Gesamtgewicht der Zubereitung (Fertigware), bzw.
      (B) 0,0001 bis 95 Gew.-%, vorzugsweise 0,001 bis 80 Gew.-%, besonders bevorzugt 0,01 bis 50 Gew.-%, bezogen auf das Gesamtgewicht der Halbfertigware,
      und
   (ii) einen oder mehrere unangenehm schmeckende Stoffe aus der Gruppe bestehend aus Aminosäuremischungen enthaltend oder bestehend aus, jeweils bezogen auf das Gewicht, 40 bis 50 Teilen L-Leucin, 20 bis 30 Teilen L-Isoleucin und 20 bis 40 Teilen L-Valin, oder 10 bis 30 Teilen L-Isoleucin, 25 bis 45 Teilen L-Leucin, 5 bis 15 Teilen L-Phenylalanin und 5 bis 15 Teilen L-Tryptophan und Peptiden mit den Sequenzen GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GW, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VW, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF oder YGLF,
      in einer Menge von
      (A) 0,001 bis 10 Gew.-%, vorzugsweise wenigstens 0,005 Gew.-%, wenigstens 0,01 Gew.-%, wenigstens 0,05 Gew.-%, wenigstens 0,1 Gew.-%, wenigstens 0,5 Gew.-% oder wenigstens 1 Gew.-%, bezogen auf das Gesamtgewicht der Zubereitung (Fertigware), bzw.
      (B) 0,001 bis 95 Gew.-%, vorzugsweise 0,01 bis 80 Gew.-%, besonders bevorzugt 0,1 bis 50 Gew.-%, bezogen auf das Gesamtgewicht der Halbfertigware.

Besonders relevant sind erfindungsgemäße Zubereitungen, zudem enthaltend einen, zwei oder mehrere weitere, nicht der Formel (I) entsprechende Substanzen zum Maskieren eines unangenehmen, insbesondere bitteren, Geschmackseindrucks eines oder mehrerer unangenehm schmeckender Stoffe.

Entsprechend der obigen Ausführungen sind erfindungsgemäße Zubereitungen bevorzugt, wobei das bzw. ein, zwei, mehrere oder sämtliche der Hydroxybenzoesäureamide der Formel (I) bzw. deren Salz(e) ausgewählt ist bzw. jeweils unabhängig voneinander ausgewählt sind aus der Gruppe bestehend aus
2,4-Dihydroxy-N-[(4-hydroxy-3-methoxy-phenyl)methyl]benzamid (Verbindung 1),
5,7-Dihydroxy-3-[(4-hydroxy-3-methoxy-phenyl)methyl]-1,3-benzoxazin-2,4-dion (Verbindung 2),
4-Hydroxy-N-[(4-hydroxy-3-methoxy-phenyl)methyl]benzamide (Verbindung 3) und
2-Hydroxy-N-[(4-hydroxy-3-methoxy-phenyl)methyl]benzamide (Verbindung 4)
und deren Salzen.

Weiterhin ist es bevorzugt, wenn für das bzw. eines, zwei, mehrere oder sämtliche Salze eines, zweier oder mehrerer verschiedener Hydroxybenzoesäureamide der Formel (I) wie oben definiert jeweils unabhängig voneinander gilt, dass das bzw. die Gegenkationen des bzw. eines, mehrerer oder sämtlicher der Salze ausgewählt ist bzw. sind aus der Gruppe bestehend aus Na⁺ und K⁺.

Für die Zwecke der hierin beschriebenen Erfindung ist es besonders bevorzugt, wenn das Gewichtsverhältnis der Gesamtmenge an Bestandteil (i) zur Gesamtmenge an Bestandteil (ii) in der Zubereitung im Bereich von 1: 100.000 bis 1 : 500 liegt, vorzugsweise im Bereich von 1: 10.000 bis 1 : 50.

Gemäß einem Aspekt der vorliegenden Erfindung ist es bevorzugt, wenn die Menge an Bestandteil (ii) ausreicht, um in einer Vergleichszubereitung, die keinen Bestandteil (i) enthält, aber ansonsten identisch zusammengesetzt ist, als unangenehmer, vorzugsweise bitterer, Geschmack wahrgenommen zu werden, und/oder wobei die Menge an Bestandteil (i) ausreicht, um im Vergleich mit der Vergleichszubereitung, die keinen Bestandteil (i) enthält, den unangenehmen, vorzugsweise bitteren, Geschmackseindruck des bzw. der unangenehm, insbesondere bitter, schmeckenden Stoffe gemäß Bestandteil (ii) zu maskieren.

Die Zubereitungen im Sinne der vorliegenden Erfindung können in Form von Kapseln, Tabletten (nichtüberzogene sowie überzogene Tabletten, z.B. magensaftresistente Überzüge), Dragees, Granulaten, Pellets, Fest-stoffmischungen, Dispersionen in flüssigen Phasen, als Emulsionen, als Pulver, als Lösungen, als Pasten oder als andere schluck- oder kaubare Zubereitungen, z.B. als Nahrungsergänzungsmittel, vorliegen. Besonders aminosäure- und peptidreiche Anwendungen sind bevorzugt, insbesondere Milchprodukte (wie Joghurts, Quarks), z.B. solche mit erhöhten Anteilen an Proteinen bzw. Aminosäuren (bis 10 Gew.-% Proteinanteil im Produkt), weiterhin Sportlernahrung (z.B. Pulver zur Zubereitung von Shakes, Riegel, Gele und andere Zubereitungsformen), z.B. solche mit einem hohen Anteil an Proteinen bzw. Aminosäuren (10-15 Gew.-% Protein- bzw. Aminosäureanteil).

Geeignete der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, eingekochte Gemüse), Knabberartikel (z.B. gebackene oder frittierte Kartoffelchips oder Kartoffelteigprodukte, Extrudate auf Mais- oder Erdnussbasis), Produkte auf Fett- und Ölbasis oder Emulsionen derselben (z.B. Mayonnaise, Remoulade, Dressings), sonstige Fertiggerichte und Suppen (z.B. Trockensuppen, Instant-Suppen, vorgegarte Suppen).

Orale pharmazeutische Zubereitungen im Sinne der vorliegenden Erfindung sind vorzugsweise Zubereitungen, die in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel, als Nahrungsergänzungsmittel, Sportlernahrung oder Medical Food verwendet werden.

Der Mundpflege dienende Zubereitungen im Sinne der vorliegenden Erfindung sind vorzugsweise Mund- und/oder Zahnpflegemittel wie Zahnpasten, Zahngele, Zahnpulver, Mundwässer, Kaugummis und andere Mundpflegemittel.

Kosmetische Zubereitungen zur Applikation im Bereich des Kopfes sind insbesondere solche, die einen unangenehm schmeckenden Stoff beinhalten und selbst bei sachgemäßer Auftragung auf die Haut mit der Mundhöhle in Kontakt treten können, also beispielsweise kosmetische Zubereitungen zur Applikation im Bereich des Kopfes wie Seifen, andere Reinigungs- oder Pflegemittel für den Gesichtsbereich, Gesichtcremes oder-lotionen oder-salben, Sonnenschutzmittel, Bartreinigungs-oder -pflegemittel, Rasierschäume, -seifen oder -gele, Lippenstifte oder andere Lippenkosmetika oder Lippenpflegemittel.

Die Zubereitungen im Sinne der vorliegenden Erfindung können auch als Halbfertigware zur Herstellung von der Ernährung oder dem Genuss dienenden Zubereitungen vorliegen, z.B. in Form von Gewürzen, Würzmischungen sowie insbesondere Aufstreuwürzungen (englisch: Seasonings), die beispielsweise im Snackbereich Anwendung finden. Auch Riech-, Aroma- oder Geschmackstoffkompositionen sind als bevorzugte Ausgestaltungen im Rahmen der vorliegenden Erfindung zu nennen.

Die hierin beschriebenen Zubereitungen können zudem weitere übliche Wirk-, Grund-, Hilfs- und Zusatzstoffe für der Ernährung, dem Genuss oder der Mundpflege dienende oder orale pharmazeutische oder kosmetische Zubereitungen im Bereich des Kopfes enthalten, insbesondere solche wie weiter unten beispielhaft aufgeführt, vorzugsweise in Mengen von 4 bis 99,999 Gew.-%, vorzugsweise 10 bis 80 Gew.-%, bezogen auf das Gesamtgewicht der Zubereitung. Ferner können die Zubereitungen Wasser in einer Menge bis zu 99,999 Gew.-%, vorzugsweise 5 bis 80 Gew.-%, bezogen auf das Gesamtgewicht der Zubereitung, enthalten.

Die erfindungsgemäßen Zubereitungen, enthaltend ein oder mehrere der erfindungsgemäß zu verwendenden Hydroxybenzoesäureamide und/oder deren Salze, werden vorzugsweise hergestellt, indem die erfindungsgemäß zu verwendenden Hydroxybenzoesäureamide und/oder deren Salze als Substanzen, als Lösung oder in Form eines Gemischs mit einem festen oder flüssigen Trägerstoff in eine der Ernährung, der Mundpflege oder dem Genuss dienende oder orale pharmazeutische oder kosmetische Basis-Zubereitung eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform werden zur Herstellung erfindungsgemäßer Zubereitungen die erfindungsgemäß zu verwendenden Hydroxybenzoesäureamide und/oder deren Salze und gegebenenfalls andere Bestandteile der erfindungsgemäßen Zubereitung auch vorher in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren werden die Hydroxybenzoesäureamide und/oder deren Salze vorher mit einem oder mehreren geeigneten Komplexbildnern, beispielsweise mit Cyclodextrinen oder Cyclodextrinderivaten, bevorzugt β-Cyclodextrin, komplexiert und in dieser komplexierten Form eingesetzt.

Besonders bevorzugt ist eine erfindungsgemäße Zubereitung, bei der die Matrix so gewählt wird, dass die Hydroxybenzoesäureamide und/oder deren Salze verzögert von der Matrix freigegeben werden, so dass man eine langanhaltende Wirkung erhält.

Als weitere Bestandteile für erfindungsgemäße Zubereitungen, insbesondere der Ernährung oder dem Genuss dienende Zubereitungen, können übliche Grund-, Hilfs- und Zusatzstoffe verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose), Zuckeralkohole (z.B. Sorbit), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nichtproteinogene Aminosäuren und verwandte Verbindungen (z.B. Taurin), weitere Peptide, native oder prozessierte Proteine (z.B. Gelatine), Enzyme (z.B. Peptidasen), Nukleinsäuren, Nucleotide, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für der Mundpflege dienende Zubereitungen), die die erfindungsgemäß zu verwendenden Hydroxybenzoesäureamide und/oder deren Salze enthalten, umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite®, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol oder Mentholderivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege dienende Zubereitungen), welche erfindungsgemäß zu verwendende Hydroxybenzoesäureamide und/oder deren Salze enthalten, umfassen im allgemeinen eine Kaugummibase, d.h. eine beim Kauen plastisch werdende Kaumasse, Zucker verschiedener Arten, Zuckeraustauschstoffe, Süßstoffe, Zuckeralkoholen, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Als Bestandteile für erfindungsgemäße orale pharmazeutische Zubereitungen können alle üblicherweise weitere Wirk-, Grund-, Hilfs- und Zusatzstoffe für orale pharmazeutische Zubereitungen verwendet werden. Als Wirkstoffe können insbesondere auch unangenehm schmeckende oral formulierbare pharmazeutische Wirkstoffe verwendet werden. Die Wirk-, Grund-, Hilfs- und Zusatzstoffe können in an sich bekannter Weise in die oralen Applikationsformen überführt werden. Dies geschieht regelmäßig unter Verwendung inerter, nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) und Geruchskorrigentien sowie nicht den bitteren Geschmack betreffende Geschmackskorrigentien.

Bevorzugt können die erfindungsgemäßen Zubereitungen auch eine Aroma-, Geschmack- oder Riechstoffkomposition enthalten, um den Geschmack und/oder Geruch der Zubereitung abzurunden und zu verfeinern. Geeignete Kompositionen enthalten z.B. synthetische, natürliche oder naturidentische Aroma-, Riech- und Geschmacksstoffe sowie geeignete Hilfs- und Trägerstoffe. Als besonders vorteilhaft wird dabei angesehen, dass ein bitterer oder metallischer Geschmackseindruck, der von in den erfindungsgemäßen Zubereitungen enthaltenen Aroma-, Riech- oder Geschmackstoffen ausgeht, maskiert werden kann und damit das gesamte sensorische Profil verbessert wird.

Erfindungsgemäße Zubereitungen, die als Halbfertigwaren vorliegen, können vorteilhafterweise zur Maskierung eines unangenehmen Geschmackseindrucks von Fertigware-Zubereitungen dienen, die unter Verwendung der Halbfertigware-Zubereitung hergestellt werden.

In einer besonders bevorzugten Ausführung der vorliegenden Erfindung werden die erfindungsgemäßen Hydroxybenzoesäureamide und/oder deren Salze in den erfindungsgemäßen Zubereitungen in Kombination mit zumindest einer weiteren Substanz zum Verändern, Maskieren oder Vermindern des unangenehmen Geschmackseindrucks eines unangenehm schmeckenden Stoffes verwendet. Auf diese Weise kann eine besonders wirksame Maskierung erreicht werden. Insbesondere die Kombination der erfindungsgemäß einzusetzenden Hydroxybenzoesäureamide bzw. deren Salzen mit anderen Geschmackskorrigenzen für unangenehme, insbesondere bittere Geschmackseindrücke ist bevorzugt. Die weiteren Geschmackskorrigenzien können beispielsweise aus der folgenden Liste ausgewählt werden: Nucleotide (z.B. Adenosin-5'-monophosphat, Cytidin-5'-monophosphat), Lactisole, Natriumsalze (z.B. Natriumchlorid, Natriumlactat, Natriumcitrat, Natriumacetat, Natriumgluconoat), Hydroxyflavanone (z.B. Eriodictyol, Homoeriodictyol oder deren Natriumsalze), Aminosäuren oder Gemische von Molkeproteinen mit Lecithinen.

Im Rahmen der vorliegenden Erfindung wird auch ein Verfahren zum (a) Maskieren des unangenehmen, insbesondere bitteren, Geschmackseindrucks eines, zweier oder mehrerer unangenehm, insbesondere bitter, schmeckender Stoffe in einer erfindungsgemäßen Zubereitung und/oder (b) Herstellen einer erfindungsgemäßen Zubereitung bereitgestellt, mit folgendem Schritt:
in Kontakt bringen oder Mischen der Bestandteile (i) und (ii) sowie optional weiterer Bestandteile
(i) ein, zwei oder mehrere verschiedene Hydroxybenzoesäureamide der Formel (I), wie oben definiert, oder ein, zwei oder mehrere verschiedene Salze eines, zweier oder mehrerer verschiedener Hydroxybenzoesäureamide der Formel (I), wie oben definiert, oder Gemisches eines, zweier oder mehrerer verschiedener Hydroxybenzoesäureamide der Formel (I), wie oben definiert, mit einem, zwei oder mehreren verschiedenen Salzen eines, zweier oder mehrerer verschiedener Hydroxybenzoesäureamide der Formel (I), wie oben definiert,
   in einer Menge von
   (A) 0,0001 bis 0,5 Gew.-%, vorzugsweise 0,001 bis 0,1 Gew.-%, bezogen auf das Gesamtgewicht der Zubereitung (Fertigware), bzw.
   (B) 0,0001 bis 95 Gew.-%, vorzugsweise 0,001 bis 80 Gew.-%, besonders bevorzugt 0,01 bis 50 Gew.-%, bezogen auf das Gesamtgewicht der Halbfertigware,
(ii) ein oder mehrere unangenehm schmeckende Stoffe aus der Gruppe bestehend aus Aminosäuremischungen enthaltend oder bestehend aus, jeweils bezogen auf das Gewicht, 40 bis 50 Teilen L-Leucin, 20 bis 30 Teilen L-Isoleucin und 20 bis 40 Teilen L-Valin, oder 10 bis 30 Teilen L-Isoleucin, 25 bis 45 Teilen L-Leucin, 5 bis 15 Teilen L-Phenylalanin und 5 bis 15 Teilen L-Tryptophan, und Peptiden mit den Sequenzen GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, W, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GW, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VW, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF oder YGLF,
   in einer Menge von
   (A) 0,001 bis 10 Gew.-%, vorzugsweise wenigstens 0,005 Gew.-%, wenigstens 0,01 Gew.-%, wenigstens 0,05 Gew.-%, wenigstens 0,1 Gew.-%, wenigstens 0,5 Gew.-% oder wenigstens 1 Gew.-%, bezogen auf das Gesamtgewicht der Zubereitung (Fertigware), bzw.
   (B) 0,001 bis 95 Gew.-%, vorzugsweise 0,01 bis 80 Gew.-%, besonders bevorzugt 0,1 bis 50 Gew.-%, bezogen auf das Gesamtgewicht der Halbfertigware.

Dabei gilt für bevorzugte Ausgestaltungen des Verfahrens das oben im Zusammenhang mit erfindungsgemäßen Zubereitungen und Verwendungen Gesagte entsprechend.

Dementsprechend ist beispielsweise ein erfindungsgemäßes Verfahren bevorzugt, wobei das bzw. ein, zwei, mehrere oder sämtliche der Hydroxybenzoesäureamide der Formel (I) bzw. deren Salz(e) ausgewählt ist bzw. jeweils unabhängig voneinander ausgewählt sind aus der Gruppe bestehend aus
2,4-Dihydroxy-N-[(4-hydroxy-3-methoxy-phenyl)methyl]benzamid (Verbindung 1),
5,7-Dihydroxy-3-[(4-hydroxy-3-methoxy-phenyl)methyl]-1,3-benzoxazin-2,4-dion (Verbindung 2),
4-Hydroxy-N-[(4-hydroxy-3-methoxy-phenyl)methyl]benzamide (Verbindung 3) und
2-Hydroxy-N-[(4-hydroxy-3-methoxy-phenyl)methyl]benzamide (Verbindung 4)
und deren Salzen.

Weiterhin gilt für das bzw. eines, zwei, mehrere oder sämtliche Salze eines, zweier oder mehrerer verschiedener Hydroxybenzoesäureamide der Formel (I) wie oben definiert vorzugsweise jeweils unabhängig voneinander, dass das bzw. die Gegenkationen des bzw. eines, mehrerer oder sämtlicher der Salze ausgewählt ist bzw. sind aus der Gruppe bestehend aus Na⁺ und K⁺.

Die Menge an Bestandteil (ii) reicht vorzugsweise aus, um in einer Vergleichszubereitung, die keinen Bestandteil (i) enthält, aber ansonsten identisch zusammengesetzt ist, als unangenehmer, vorzugsweise bitterer, Geschmack wahrgenommen zu werden, und/oder die Menge an Bestandteil (i) reicht vorzugsweise aus, um im Vergleich mit der Vergleichszubereitung, die keinen Bestandteil (i) enthält, den unangenehmen, vorzugsweise bitteren, Geschmackseindruck des bzw. der unangenehm, insbesondere bitter, schmeckenden Stoffe gemäß Bestandteil (ii) zu maskieren.

Im Folgenden wird die vorliegende Erfindung anhand ausgewählter Beispiele näher verdeutlicht. Die Beispiele dienen daher nur zur Verdeutlichung der Erfindung, ohne diese damit einzuschränken.

### Beispiele

### Beispiel 1: Wirkung von

2,4-Dihydroxy-N-[(4-hydroxy-3-methoxy-phenyl)methyl]benzamid (Verbindung 1),5,7-Dihydroxy-3-[(4-hydroxy-3-methoxy-phenyl)methyl]-1,3-benzoxazin-2,4-dion (Verbindung 2), 4-Hydroxy-N-[(4-hydroxy-3-methoxy-phenyl)methyl]benzamide (Verbindung 3), 2-Hydroxy-N-[(4-hydroxy-3-methoxy-phenyl)methyl]benzamide (Verbindung 4) und N-[(4-hydroxy-3-methoxy-phenyl)methyl]-2,4-dimethoxy-benzamide (Verbindung 5, Vergleichsbeispiel) auf die Bitterkeit eines Gemischs von Aminosäuren

Die maskierende Wirkung erfindungsgemäß bevorzugt zu verwendender Hydroxybenzoesäureamide (Verbindungen 1-4) sowie einer Vergleichssubstanz (Verbindung 5) auf eine Mischung von bitteren Aminosäuren (2 g/l L-Isoleucin; 3,5 g/l L-Leucin; 1 g/l L-Phenylalanin; 0,7 g/l L-Tryptophan in Wasser) wurde durch ein Expertenpanel (n=13) bewertet (Einstufung 0 [nicht bitter] bis 10 [extrem bitter]). Die Verkostung erfolgte mit Nasenklammer, um olfaktorische Einflüsse auszuschliessen, in randomisierten Duo-Tests. Die Bitterkeit der Aminosäuremischung wurde vom Panel mit 4,98±0,31 auf einer Skala von 0-10 bewertet.

| Probe | Bittergeschmack Aminosäuremisch ung | Bittergeschmack Aminosäuremisch ung + Probe | Bitterreduktion in % gegenüber der Aminosäuremisch ung |
|---|---|---|---|
| 100 ppm 2,4-Dihydroxy-N-[(4-hydroxy-3-methoxyphenyl)methyl]benza mid (Verbindung 1) | 5.27 | 3.90 | -25,9 |
| 100 ppm 5,7-Di hyd roxy-3-[(4-hydroxy-3-methoxyphenyl)methyl]-1,3-benzoxazin-2,4-dion (Verbindung 2) | 4.59 | 3.20 | -30,3 |
| 100 ppm 4-Hydroxy-N-[(4-hyd roxy-3-methoxyphenyl)methyl]benza mide (Verbindung 3) | 4.82 | 4.11 | -14.1 |
| 100 ppm 2-Hydroxy-N-[(4-hyd roxy-3-methoxyphenyl)methyl]benza mide (Verbindung 4) | 5.13 | 4.74 | -7.7 |
| 100 ppm N-[(4-hydroxy-3-methoxyphenyl)methyl]-2,4-dimethoxybenzamide (Verbindung 5, Vergleichsbeispiel) | 5.59 | 6.86 | 22.8 |

### Beispiel 2: Wirkung von 2,4-Dihydroxy-N-[(4-hydroxy-3-methoxyphenyl)methyl]benzamid (Verbindung 1) und N-[(4-hydroxy-3-methoxyphenyl)methyl]-2,4-dimethoxy-benzamide (Verbindung 5, Vergleichsbeispiel) auf den bitteren Geschmack von Peptiden

Die maskierende Wirkung von 2,4-Dihydroxy-N-[(4-hydroxy-3-methoxyphenyl)methyl]benzamid (Verbindung 1) und N-[(4-hydroxy-3-methoxyphenyl)methyl]-2,4-dimethoxy-benzamide (Verbindung 5) auf den Bittergeschmack von Peptiden wurde in einem Expertenpanel (n=15) in randomisierten Duo-Tests untersucht. Dazu Lösungen bitterer Peptide in Wasser mit oder ohne Zusatz der beispielhaften Substanzen verkostet und der wahrgenommene Bittergeschmack auf einer Skala von 0 (nicht bitter) bis 10 (extrem bitter) bewertet.

Mit Verbindung 1 konnte dabei jeweils eine deutliche Reduktion der Bitterkeit für eine Vielzahl von Peptiden erzielt werden (mit Ausnahme des Dipeptids Leu-Trp (Bestätigung der Daten von Ley et al. (Ley et al., J Agr Food Chem 54, 8574-8579 (2006)) sowie des Tripeptids Glu-Leu-Leu, die hier jeweils als Vergleichsbeispiele mit aufgenommen sind) (s. Tabelle). Verbindung 5 ((weiteres) Vergleichsbeispiel) war bei keinem der Peptide in der Lage, eine Reduktion der Bitterkeit zu erzielen, teilweise verstärkte Verbindung 5 den Bittergeschmack sogar.

| Peptid | Bittergesch mack Peptidlösun g | Bittergesch mack Peptidlösun g + 100 ppm (Verbindun g 1) | Bitterredukti on Verbindung 1 (%) | Bittergesch mack Peptidlösun g + 100 ppm Verbindung 5 (Vergleichs beispiel) | Bitterredukti on Verbindung 5 (%) (Vergleichs beispiel) |
|---|---|---|---|---|---|
| Arg-Pro 0.1% (w/v) | 1,85 | 1,47 | -20,9 | 2,05 | 10,81 |
| Trp-Trp-Trp 0.01 % (w/v) | 6,47 | 5,35 | -17,4 | 6,40 | -1,08 |
| Tyr-Pro-Phe-Pro 0.1% (w/v) | 4,48 | 3,43 | -23,3 | 4,68 | 4,46 |
| Leu-Trp 0.2% (w/v) (Vergleichs beispiel) | 6.8 | 6.5 | -4 | 7,1 | 4,41 |
| Glu-Leu-Leu 0.2% (w/v) (Vergleichs beispiel) | 5.3 | 5.1 | -3,77 | 5,6 | 5,66 |

### Beispiel 3: Wirkung von 2,4-Dihydroxy-N-[(4-hydroxy-3-methoxyphenyl)methyl]benzamid (Verbindung 1) auf den bitteren Geschmack einer Lösung von Molkeproteinhydrolysat.

Die bittermaskierende Wirkung von 2,4-dihydroxy-N-[(4-hydroxy-3-methoxyphenyl)methyl]benzamid (Verbindung 1) auf den bitteren Geschmack von Molkeproteinhydrolysat wurde in einem Expertenpanel [n=8] auf einer Lösung von 3% (w/v) eines kommerziell erhältlichen Molkeproteinhydrolysats (BioZate 3 der Fima Davisco) in randomisierten Duotests getestet. Der wahrgenommene Bittergeschmack wurde dabei auf einer Skala von 0 [nicht bitter] bis 10 [extrem bitter] eingestuft. Zum Vergleich wurde eine Kombination der Verbindung 1 mit einem herkömmlichen Bittermasking-Aroma getestet, um mögliche additive Effekte zu untersuchen.

| Probe | Reduktion des Bittergeschmacks [%] |
|---|---|
| Base [Molkeproteinhydrolysat (3% (w/v) in Wasser)] | 0 |
| Base + 100 ppm Verbindung 1 | -24,5 |
| Base + Bittermaskingaroma (Symrise) (1,6% (w/v)) + 100 ppm Verbindung 1 | -65,5 |

### Anwendungsbeispiel 1: Sprühgetrocknete Zubereitung als Halbfertigware zur Aromatisierung von erfindungsgemäßen Fertigwaren

| **Inhaltsstoff** | **Einsatz in Gew.-%** |
|---|---|
| Trinkwasser | 60,8 % |
| Maltodextrin aus Weizen | 24,3 % |
| Gummi Arabicum | 6,1 % |
| 2,4-Dihydroxy-N-[(4-hydroxy-3-methoxyphenyl)methyl]benzamid (Verbindung 1) | 8,8 % |

Das Trinkwasser wird in einem Behälter vorgelegt und das Maltodextrin und das Gummi Arabicum darin gelöst. Anschließend wird das 2,4-dihydroxy-N-[(4-hydroxy-3-methoxy-phenyl)methyl]benzamid (Verbindung 1) mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 - 195°C, Solltemperatur Ausgang: 70 - 75°C).

Die sprühgetrocknete Halbfertigware enthält ca. 18 - 22 % an Verbindung 1 und kann zur Herstellung einer hierin beschriebenen erfindungsgemäßen Zubereitung verwendet werden.

### Anwendungsbeispiel 2: Verwendung in einem Soja-Getränk

Die Verbindung 2,4-Dihydroxybenzoesäure-*N*-(4-hydroxy-3-methoxybenzyl)amid (Verbindung 1) wurde in Ethanol vorgelöst und zu einer Sojamilch aus einem lokalen Supermarkt, welche unangenehm schmeckende Stoffe gemäß Anspruch 1 enthält, hinzugefügt. Die Mischung wurde zusammen mit dem Milcharoma im Becherglas verrührt.

| **Inhaltsstoff** | **Einsatz in Gew.-%** |
|---|---|
| Sojamilch (lokaler Supermarkt, enthält durchschnittlich ca. 15% freie Aminosäuren) | 99,8 % |
| Milcharoma (Symrise) | 0,1 % |
| 10 % 2,4-Dihydroxybenzoesäure-*N*-(4-hydroxy-3-methoxybenzyl)amid (Verbindung1) in Ethanol | 0,1 % |

### Anwendungsbeispiel 3: Verwendung in einem Soja-Getränk in Kombination mit γ-Aminobuttersäure

γ-Aminobuttersäure wurde in Wasser und 2,4-Dihydroxybenzoesäure-*N*-(4-hydroxy-3-methoxybenzyl)amid in Ethanol vorgelöst und gemäß Tabelle zu einer Sojamilch aus einem lokalen Supermarkt, welche unangenehm schmeckende Stoffe gemäß Anspruch 1 enthält, hinzugefügt (Mischungen A-C). Die Mischungen wurden zusammen mit dem Milcharoma im Becherglas verrührt.

| **Inhaltsstoff** | **Einsatz in Gew.-%** | | |
|---|---|---|---|
| | **A** | **B** | **C** |
| Sojamilch (lokaler Supermarkt) | zu 100% auffüllen | zu 100% auffüllen | zu 100% auffüllen |
| Milcharoma (Symrise) | 0,1 % | 0,1 % | 0,1 % |
| 10 % 2,4-Dihydroxybenzoesäure-*N*-(4-hydroxy-3-methoxybenzyl)amid in Ethanol | 0,1 % | - | 0,1% |
| 1 % γ-Aminobuttersäure in Wasser | 0,1 % | - | - |

Verglichen mit Zubereitung B waren die Zubereitungen A und C deutlich weniger bitter. In Zubereitung A war zudem die Adstringenz und die bohnige Note wesentlich reduziert.

### Anwendungsbeispiel 4: Molkenproteindrink

Herstellung eines Frucht-Molkenproteingetränkes nach u.g. Rezeptur mit anschließender Homogenisierung und Pasteurisierung.

| **Inhaltsstoff** | **Einsatz in Gew.-%** | |
|---|---|---|
| | **A** | **B** |
| Molkenproteinisolat (min. 88% Protein TS, enthält unangenehm schmeckende Stoffe gemäß Anspruch 1) | 8 % | 8% |
| Fruchtsaftkonzentrat-Mix (Mango, Banane, Karotte, Orange) | 10 % | 10% |
| Zitronensäure 50%ig | 0,2 % | 0,2 % |
| Pektin | 0,4 % | 0,4 % |
| Vitamin-Mischung | 0,01 % | 0,01 % |
| Mineralsalz-Mischung | 1 % | 1 % |
| 10 % 2,4-Dihydroxybenzoesäure-*N*-(4-hydroxy-3-methoxybenzyl)amid in Ethanol | - | 0,1 % |
| Mango Aroma (Symrise) | 0,05 % | 0,05 % |
| Trinkwasser | zu 100% auffüllen | zu 100% auffüllen |

Verglichen mit der Vergleichszubereitung A war die Zubereitungen B deutlich weniger bitter.

### Anwendungsbeispiel 5: Hochproteinhaltiges Getränk (Sportlernahrung)

Die trockenen Zutaten werden gemischt und anschließend in Trinkwasser gelöst. In Beispiel C wird zum Schluss die 10 %ige Lösung des 2,4-Dihydroxybenzoesäure-*N-*(4-hydroxy-3-methoxybenzyl)amid zugegeben und verrührt.

| **Inhaltsstoff** | **Einsatz in Gew.-%** | | |
|---|---|---|---|
| | **A** | **B** | **C** |
| Molkenproteinisolat (min. 88% Protein TS, enthält unangenehm schmeckende Stoffe gemäß Anspruch 1) | 8 % | 8 % | 8 % |
| Aminosäuremischung (45 % L-Leucin; 30% L-Valin, 25% L-Isoleucin) | 1 % | 1 % | 1 % |
| Zitronensäure | 0,1 % | 0,1 % | 0,1 % |
| Sucralose | 0,007 % | 0,007 % | 0,007 % |
| Orangen Trockenaroma (Symrise) | 0,1 % | 0,1 % | 0,1 % |
| Bittermasking Trockenaroma (Symrise) | - | 1,6 % | 1,6 % |
| 10 % 2,4-Dihydroxybenzoesäure-*N*-(4-hydroxy-3-methoxybenzyl)amid in Ethanol | - | - | 0,1 % |
| Trinkwasser | zu 100% auffüllen | zu 100% auffüllen | zu 100% auffüllen |

Verglichen mit der Vergleichszubereitung A sind die Zubereitungen B und C weniger bitter. Die Zubereitung C ist zudem deutlich weniger bitter als die Zubereitung B.

### Anwendungsbeispiel 6: Diätetisches Lebensmittel für besondere medizinische Zwecke (FSMP)

Herstellung eines UHT-Getränkes nach u.g. Rezeptur.

| **Inhaltsstoff** | **Einsatz in Gew.-%** | |
|---|---|---|
| | **A** | **B** |
| Molkenproteinkonzentrat (min. 80% Protein TS, enthält unangenehm schmeckende Stoffe gemäß Anspruch 1) | 12 % | 12 % |
| Trinkwasser | zu 100% auffüllen | zu 100% auffüllen |
| Glukosesirup | 10 % | 10% |
| Pflanzliche Öle | 4 % | 4% |
| Saccharose | 2 % | 2 % |
| Maltodextrin | 1,5 % | 1,5 % |
| Vitamin-Mischung | 0,05 % | 0,05 % |
| Mineralsalz-Mischung | 2 % | 2 % |
| Vanille Aroma (Symrise) | 0,2 % | 0,2 % |
| Lecithin | 0,1 % | 0,1 % |
| Zitronensäure | 0,05 % | 0,05 % |
| 10 % 2,4-Dihydroxybenzoesäure-*N*-(4-hydroxy-3-methoxybenzyl)amid in Ethanol | - | 0,1 % |

Die Zubereitung B ist deutlich weniger bitter und austrocknend im Nachgeschmack als die Vergleichszubereitung A.

### Anwendungsbeispiel 7: Proteinriegel

Die Cerealien und das Molkenproteinisolat werden gemischt. Aus den restlichen Zutaten (ohne Aroma und 2,4-Dihydroxybenzoesäure-*N*-(4-hydroxy-3-methoxybenzyl)amid) wird ein Sirup gekocht. Am Ende der Kochzeit wird das Aroma bzw. die 10%ige Lösung 2,4-Dihydroxybenzoesäure-*N*-(4-hydroxy-3-methoxybenzyl)amid in Ethanol zugegeben. Der Sirup wird mit den Cerealien gemischt und zu Riegeln geformt.

| **Inhaltsstoff** | **Einsatz in Gew.-%** | | |
|---|---|---|---|
| | **A** | **B** | **C** |
| Haferflocken | 13 % | 13 % | 13 % |
| Reis-Crispies | 14 % | 14 % | 14 % |
| Corn Flakes | 10 % | 10 % | 10 % |
| Molkenproteinisolat (min. 88% Protein TS, enthält unangenehm schmeckende Stoffe gemäß Anspruch 1) | 10 % | 10 % | 10 % |
| Glucose Sirup | 0,1 % | 0,1 % | 0,1 % |
| Saccharose | 12 % | 12 % | 12 % |
| Glycerin | 1 % | 1 % | 1 % |
| Salz | 0,2 % | 0,2 % | 0,2 % |
| Trinkwasser | 13,5 % | 13,5 % | 13,5 % |
| Pflanzenfett | 10 % | 10 % | 10 % |
| Lecithin | 0,2 % | 0,2 % | 0,2 % |
| Bittermasking Aroma (Symrise) | - | - | 0,3 % |
| Karamel Aroma (Symrise) | 0,1 % | 0,1 % | 0,1 % |
| 10 % 2,4-Dihydroxybenzoesäure-*N*-(4-hydroxy-3-methoxybenzyl)amid in Ethanol | | 0,1 % | 0,1 % |

Verglichen mit der Vergleichszubereitung A sind die Zubereitungen B und C weniger bitter. Die Zubereitung C ist zudem deutlich weniger austrocknend und adstringierend im Nachgeschmack als die Zubereitung B.

### Anwendungsbeispiel 8: Hochproteinhaltiges Milchprodukt (Quark)

2,4-Dihydroxybenzoesäure-*N*-(4-hydroxy-3-methoxybenzyl)amid wird in Ethanol vorgelöst und mit dem Sahne-Aroma in Quark eingerührt. In die Vergleichszubereitung wird nur das Sahne-Aroma eingerührt.

| **Inhaltsstoff** | **Einsatz in Gew.-%** | | |
|---|---|---|---|
| | **A** | **B** | **C** |
| Hochproteinhaltiges Marktprodukt (enthält unangenehm schmeckende Stoffe gemäß Anspruch 1); (französischer Markt) (9% Protein, 2,9% Fett) | zu 100% auffüllen | zu 100% auffüllen | zu 100% auffüllen |
| Saccharose | 4 % | 4 % | 4 % |
| Sahnearoma (Symrise) | 0,15 % | 0,15 % | 0,15 % |
| 10 % 2,4-Dihydroxybenzoesäure-*N*-(4-hydroxy-3-methoxybenzyl)amid in Ethanol | - | 0,05 % | 0,1% |

Verglichen mit Zubereitung A waren die Zubereitungen B und C deutlich weniger bitter und adstringierend. In Zubereitung C war die Adstringenz noch deutlicher reduziert.

## Patentansprüche

1. Verwendung eines, zweier oder mehrerer verschiedener Hydroxybenzoesäureamide der Formel (I) 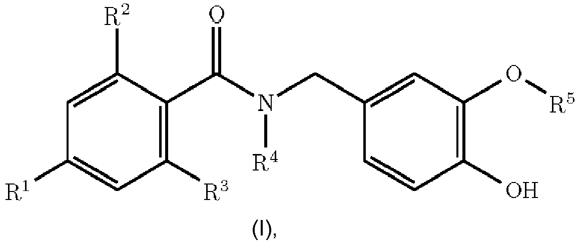 wobei
R¹ und R² unabhängig voneinander Wasserstoff, Hydroxy oder Methoxy bedeuten, mit der Maßgabe, dass mindestens einer der Reste R¹ oder R² Hydroxy bedeutet,
und
entweder
R³ Wasserstoff oder Hydroxy
und
R⁴ Wasserstoff bedeutet
oder
R³ und R⁴
gemeinsam eine Gruppe -O-C(=O)- darstellen und damit einen Ring in Form eines cyclischen Carbamats bilden,
und
R⁵ Wasserstoff oder Methyl bedeutet,
oder
eines, zweier oder mehrerer verschiedener Salze eines, zweier oder mehrerer verschiedener Hydroxybenzoesäureamide der Formel (I) wie oben definiert
oder
eines Gemisches eines, zweier oder mehrerer verschiedener Hydroxybenzoesäureamide der Formel (I) wie oben definiert mit einem, zwei oder mehreren verschiedenen Salzen eines, zweier oder mehrerer verschiedener Hydroxybenzoesäureamide der Formel (I) wie oben definiert
zum Maskieren eines unangenehmen Geschmackseindrucks, vorzugsweise eines bitteren, adstringierenden, pappigen, staubigen, trockenen, mehligen, ranzigen und/oder metallischen Geschmacks oder Nachgeschmacks, eines oder mehrerer unangenehm schmeckender Stoffe, wobei der bzw. einer, mehrere oder sämtliche der unangenehmen Stoffe ausgewählt ist bzw. sind aus der Gruppe bestehend aus Aminosäuremischungen enthaltend oder bestehend aus, jeweils bezogen auf das Gewicht, 40 bis 50 Teilen L-Leucin, 20 bis 30 Teilen L-Isoleucin und 20 bis 40 Teilen L-Valin, oder 10 bis 30 Teilen L-Isoleucin, 25 bis 45 Teilen L-Leucin, 5 bis 15 Teilen L-Phenylalanin und 5 bis 15 Teilen L-Tryptophan, und Peptiden mit den Sequenzen GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VW, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF oder YGLF.

2. Verwendung nach Anspruch 1, wobei das bzw. ein, zwei, mehrere oder sämtliche der eingesetzten Hydroxybenzoesäureamide der Formel (I) bzw. deren Salz(e) ausgewählt ist bzw. jeweils unabhängig voneinander ausgewählt sind aus der Gruppe bestehend aus
2,4-Dihydroxy-N-[(4-hydroxy-3-methoxy-phenyl)methyl]benzamid (Verbindung 1),
5,7-Dihydroxy-3-[(4-hydroxy-3-methoxy-phenyl)methyl]-1,3-benzoxazin-2,4-dion (Verbindung 2),
4-Hydroxy-N-[(4-hydroxy-3-methoxy-phenyl)methyl]benzamide (Verbindung 3) und
2-Hydroxy-N-[(4-hydroxy-3-methoxy-phenyl)methyl]benzamide (Verbindung 4)
und deren Salzen.

3. Verwendung nach einem der vorangehenden Ansprüche, in Kombination mit einer, zwei oder mehreren weiteren Substanzen zum Maskieren eines unangenehmen, insbesondere bitteren, Geschmackseindrucks eines oder mehrerer unangenehm schmeckender Stoffe.

4. Verwendung nach einem der vorangehenden Ansprüche in einer Zubereitung ausgewählt aus der Gruppe bestehend aus
- der Ernährung oder der Mundpflege dienenden, oralen pharmazeutischen oder kosmetischen, zur Applikation im Bereich des Kopfes dienenden Zubereitungen (Fertigwaren), und
- Halbfertigwaren, insbesondere solchen zur Herstellung einer der Ernährung oder der Mundpflege dienenden, oralen pharmazeutischen oder kosmetischen, zur Applikation im Bereich des Kopfes dienenden Zubereitung (Fertigwaren), vorzugsweise in Form einer Riech-, Aroma- oder Geschmackstoffkomposition oder Würzmischung.

5. Zubereitung ausgewählt aus der Gruppe bestehend aus
(A) der Ernährung, dem Genuss oder der Mundpflege dienenden, oralen pharmazeutischen oder kosmetischen, zur Applikation im Bereich des Kopfes dienenden Zubereitungen (Fertigwaren), und
(B) Halbfertigwaren, insbesondere solchen zur Herstellung einer der Ernährung, dem Genuss oder der Mundpflege dienenden, oralen pharmazeutischen oder kosmetischen, zur Applikation im Bereich des Kopfes dienenden Zubereitung (Fertigwaren), vorzugsweise in Form einer Riech-, Aroma- oder Geschmackstoffkomposition oder Würzmischung,
enthaltend
(i) eine, zwei oder mehrere verschiedene Hydroxybenzoesäureamide der Formel (I), wie in einem der Ansprüche 1 bis 3 definiert, oder ein, zwei oder mehrere verschiedene Salze eines, zweier oder mehrerer verschiedener Hydroxybenzoesäureamide der Formel (I), wie in einem der Ansprüche 1 bis 3 definiert, oder ein Gemisches eines, zweier oder mehrerer verschiedener Hydroxybenzoesäureamide der Formel (I), wie in einem der Ansprüche 1 bis 3 definiert, mit einem, zwei oder mehreren verschiedenen Salzen eines, zweier oder mehrerer verschiedener Hydroxybenzoesäureamide der Formel (I), wie in einem der Ansprüche 1 bis 3 definiert,
in einer Menge von
(A) 0,0001 bis 0,5 Gew.-%, vorzugsweise 0,001 bis 0,1 Gew.-%, bezogen auf das Gesamtgewicht der Zubereitung (Fertigware), bzw.
(B) 0,0001 bis 95 Gew.-%, vorzugsweise 0,001 bis 80 Gew.-%, besonders bevorzugt 0,01 bis 50 Gew.-%, bezogen auf das Gesamtgewicht der Halbfertigware,
und
(ii) einen oder mehrere unangenehm schmeckende Stoffe aus der Gruppe bestehend aus Aminosäuremischungen enthaltend oder bestehend aus, jeweils bezogen auf das Gewicht, 40 bis 50 Teilen L-Leucin, 20 bis 30 Teilen L-Isoleucin und 20 bis 40 Teilen L-Valin, oder 10 bis 30 Teilen L-Isoleucin, 25 bis 45 Teilen L-Leucin, 5 bis 15 Teilen L-Phenylalanin und 5 bis 15 Teilen L-Tryptophan, und Peptiden mit den Sequenzen GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VVV, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF oder YGLF,
in einer Menge von
(A) 0,001 bis 10 Gew.-%, vorzugsweise wenigstens 0,005 Gew.-%, wenigstens 0,01 Gew.-%, wenigstens 0,05 Gew.-%, wenigstens 0,1 Gew.-%, wenigstens 0,5 Gew.-% oder wenigstens 1 Gew.-%, bezogen auf das Gesamtgewicht der Zubereitung (Fertigware), bzw.
(B) 0,001 bis 95 Gew.-%, vorzugsweise 0,01 bis 80 Gew.-%, besonders bevorzugt 0,1 bis 50 Gew.-%, bezogen auf das Gesamtgewicht der Halbfertigware.

6. Zubereitung nach Anspruch 5, zudem enthaltend einen, zwei oder mehrere weitere, nicht der Formel (I) entsprechende Substanzen zum Maskieren eines unangenehmen, insbesondere bitteren, Geschmackseindrucks eines oder mehrerer unangenehm schmeckender Stoffe.

7. Zubereitung nach Anspruch 5 oder 6, wobei das bzw. ein, zwei, mehrere oder sämtliche der Hydroxybenzoesäureamide der Formel (I) bzw. deren Salz(e) ausgewählt ist bzw. jeweils unabhängig voneinander ausgewählt sind aus der Gruppe bestehend aus
2,4-Dihydroxy-N-[(4-hydroxy-3-methoxy-phenyl)methyl]benzamid (Verbindung 1),
5,7-Dihydroxy-3-[(4-hydroxy-3-methoxy-phenyl)methyl]-1,3-benzoxazin-2,4-dion (Verbindung 2),
4-Hydroxy-N-[(4-hydroxy-3-methoxy-phenyl)methyl]benzamide (Verbindung 3) und
2-Hydroxy-N-[(4-hydroxy-3-methoxy-phenyl)methyl]benzamide (Verbindung 4)
und deren Salzen.

8. Zubereitung nach einem der Ansprüche 5 bis 7, wobei für das bzw. eines, zwei, mehrere oder sämtliche Salze eines, zweier oder mehrerer verschiedener Hydroxybenzoesäureamide der Formel (I) wie in einen der vorhergehenden Ansprüche definiert jeweils unabhängig voneinander gilt, dass das bzw. die Gegenkationen des bzw. eines, mehrerer oder sämtlicher der Salze ausgewählt ist bzw. sind aus der Gruppe bestehend aus Na⁺ und K⁺.

9. Zubereitung nach einem der Ansprüche 5 bis 8, wobei das Gewichtsverhältnis der Gesamtmenge an Bestandteil (i) zur Gesamtmenge an Bestandteil (ii) in der Zubereitung im Bereich von 1 : 100.000 bis 1 : 500 liegt, vorzugsweise im Bereich von 1: 10.000 bis 1 : 50.

10. Zubereitung nach einem der Ansprüche 5 bis 9, wobei die Menge an Bestandteil (ii) ausreicht, um in einer Vergleichszubereitung, die keinen Bestandteil (i) enthält, aber ansonsten identisch zusammengesetzt ist, als unangenehmer, vorzugsweise bitterer, Geschmack wahrgenommen zu werden, und/oder wobei die Menge an Bestandteil (i) ausreicht, um im Vergleich mit der Vergleichszubereitung, die keinen Bestandteil (i) enthält, den unangenehmen, vorzugsweise bitteren, Geschmackseindruck des bzw. der unangenehm, insbesondere bitter, schmeckenden Stoffe gemäß Bestandteil (ii) zu maskieren.

11. Verfahren zum (a) Maskieren des unangenehmen, insbesondere bitteren, Geschmackseindrucks eines, zweier oder mehrerer unangenehm, insbesondere bitter, schmeckender Stoffe in einer Zubereitung nach einem der Ansprüche 5 bis 10 und/oder (b) Herstellen einer Zubereitung nach einem der Ansprüche 5 bis 10, mit folgendem Schritt:
in Kontakt bringen oder Mischen der Bestandteile (i) und (ii) sowie optional weiterer Bestandteile
(i) ein, zwei oder mehrere verschiedene Hydroxybenzoesäureamide der Formel (I), wie in einem der Ansprüche 1 bis 3 definiert, oder ein, zwei oder mehrere verschiedene Salze eines, zweier oder mehrerer verschiedener Hydroxybenzoesäureamide der Formel (I), wie in einem der Ansprüche 1 bis 3 definiert, oder Gemisches eines, zweier oder mehrerer verschiedener Hydroxybenzoesäureamide der Formel (I), wie in einem der Ansprüche 1 bis 3 definiert, mit einem, zwei oder mehreren verschiedenen Salzen eines, zweier oder mehrerer verschiedener Hydroxybenzoesäureamide der Formel (I), wie in einem der Ansprüche 1 bis 3 definiert,
in einer Menge von
(A) 0,0001 bis 0,5 Gew.-%, vorzugsweise 0,001 bis 0,1 Gew.-%, bezogen auf das Gesamtgewicht der Zubereitung (Fertigware), bzw.
(B) 0,0001 bis 95 Gew.-%, vorzugsweise 0,001 bis 80 Gew.-%, besonders bevorzugt 0,01 bis 50 Gew.-%, bezogen auf das Gesamtgewicht der Halbfertigware,
(ii) ein oder mehrere unangenehm schmeckende Stoffe aus der Gruppe bestehend aus Aminosäuremischungen enthaltend oder bestehend aus, jeweils bezogen auf das Gewicht, 40 bis 50 Teilen L-Leucin, 20 bis 30 Teilen L-Isoleucin und 20 bis 40 Teilen L-Valin, oder 10 bis 30 Teilen L-Isoleucin, 25 bis 45 Teilen L-Leucin, 5 bis 15 Teilen L-Phenylalanin und 5 bis 15 Teilen L-Tryptophan, und Peptiden mit den Sequenzen GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VVV, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF oder YGLF,
in einer Menge von
(A) 0,001 bis 10 Gew.-%, vorzugsweise wenigstens 0,005 Gew.-%, wenigstens 0,01 Gew.-%, wenigstens 0,05 Gew.-%, wenigstens 0,1 Gew.-%, wenigstens 0,5 Gew.-% oder wenigstens 1 Gew.-%, bezogen auf das Gesamtgewicht der Zubereitung (Fertigware), bzw.
(B) 0,001 bis 95 Gew.-%, vorzugsweise 0,01 bis 80 Gew.-%, besonders bevorzugt 0,1 bis 50 Gew.-%, bezogen auf das Gesamtgewicht der Halbfertigware.

12. Verfahren nach Anspruch 11, wobei das bzw. ein, zwei, mehrere oder sämtliche der Hydroxybenzoesäureamide der Formel (I) bzw. deren Salz(e) ausgewählt ist bzw. jeweils unabhängig voneinander ausgewählt sind aus der Gruppe bestehend aus
2,4-Dihydroxy-N-[(4-hydroxy-3-methoxy-phenyl)methyl]benzamid (Verbindung 1),
5,7-Dihydroxy-3-[(4-hydroxy-3-methoxy-phenyl)methyl]-1 ,3-benzoxazin-2,4-dion (Verbindung 2),
4-Hydroxy-N-[(4-hydroxy-3-methoxy-phenyl)methyl]benzamide (Verbindung 3) und
2-Hydroxy-N-[(4-hydroxy-3-methoxy-phenyl)methyl]benzamide (Verbindung 4)
und deren Salzen.

13. Verfahren nach Anspruch 11 oder 12, wobei für das bzw. eines, zwei, mehrere oder sämtliche Salze eines, zweier oder mehrerer verschiedener Hydroxybenzoesäureamide der Formel (I) wie in einen der vorhergehenden Ansprüche definiert jeweils unabhängig voneinander gilt, dass das bzw. die Gegenkationen des bzw. eines, mehrerer oder sämtlicher der Salze ausgewählt ist bzw. sind aus der Gruppe bestehend aus Na⁺ und K⁺.

## Claims

1. Use of one, two or more different hydroxybenzoic acid amides of formula (I) 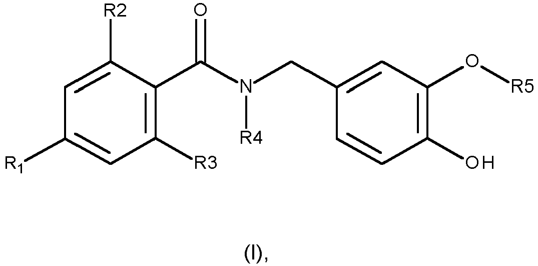 wherein
R¹ and R² denote independently of each other hydrogen, hydroxyl or methoxy, with the provision that at least one of the groups R¹ or R² denote hydroxyl,
and
either
R³ denotes hydrogen or hydroxyl
and
R⁴ denotes hydrogen
or
R³ and R⁴ together denote a group -O-C(=O)- and thus form a ring in form of a cyclic carbamate,
and
R⁵ denotes hydrogen or methyl,
or
of one, two or more different salts of one, two or more different hydroxybenzoic acid amides of formula (I) as defined above
or
of a mixture of one, two or more different hydroxybenzoic acid amides of formula (I), as defined above, with one, two or more different salts of one, two or more different hydroxybenzoic acid amides of formula (I) as defined above
for masking an unpleasant taste impression, preferably a bitter, astringent, cloggy, dusty, dry, floury, rancid and/or metallic taste or aftertaste, of one or more unpleasant tasting substances, wherein the or, respectively, one, more or all of the unpleasant tastes is/are selected from the group consisting of amino acid mixtures comprising or consisting of, each related to the weight, 40 to 50 parts L-leucine, 20 to 30 parts L-isoleucine and 20 to 40 parts L-valine or 10 to 30 parts L-isoleucine, 25 to 45 parts L-leucine, 5 to 15 parts L-phenylalanine and 5 to15 parts L-tryptophan, and peptides with the sequences GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VVV, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF or YGLF.

2. Use according to claim 1, wherein the or, respectively, one, two, more or all of the used hydroxybenzoic acid amides of formula (I) or, respectively, their salt(s) is/are independently of each other selected from the group consisting of
2,4-Dihydroxy-N-[(4-hydroxy-3-methoxy-phenyl)methyl]benzamide (compound 1),
5,7-Dihydroxy-3-[(4-hydroxy-3-methoxy-phenyl)methyl]-1,3-benzoxazin-2,4-dione (compound 2),
4-Hydroxy-N-[(4-hydroxy-3-methoxy-phenyl)methyl]benzamide (compound 3) and
2-Hydroxy-N-[(4-hydroxy-3-methoxy-phenyl)methyl]benzamide (compound 4)
and their salts.

3. Use according to one of the preceding claims, in combination with one, two or more further substances for masking an unpleasant, particularly bitter, taste impression of one or more unpleasant tasting substances.

4. Use according to one of the preceding claims in a preparation selected from the group consisting of
- oral pharmaceutical or cosmetic preparations (finished product) serving for nutrition or oral care and for the application in the head area, and
- semi-finished products, particularly such serving for the production of oral pharmaceutical or cosmetic preparations (finished product) serving for nutrition or oral care and for the application in the head area, preferably in form of an olfactory, aromatic or flavour composition or seasoning mix.

5. Composition selected from the group consisting of
(A) oral pharmaceutical or cosmetic preparations (finished product) serving for nutrition or oral care and for the application in the head area, and
(B) semi-finished products, particularly such serving for the production of oral pharmaceutical or cosmetic preparations (finished product) serving for nutrition or oral care and for the application in the head area, preferably in form of a olfactory, aromatic or flavour composition or seasoning mix,
comprising
(i) one, two or more different hydroxybenzoic acid amides of formula (I), as defined in one of the claims 1 to 3, or one, two or more different salts of one, two or more different hydroxybenzoic acid amides of formula (I), as defined in one of the claims 1 to 3, or a mixture of one, two or more different hydroxybenzoic acid amides of formula (I), as defined in one of the claims 1 to 3, with one, two or more different salts of one, two or more different hydroxybenzoic acid amides of formula (I), as defined in one of the claims 1 to 3,
in an amount of
(A) 0.0001 to 0.5 wt.-%, preferably 0.001 to 0.1 wt.-%, related to the total weight of the composition (finished product), or, respectively,
(B) 0.0001 to 95 wt.-%, preferably 0.001 to 80 wt.-%, particularly preferably 0.01 to 50 wt.-%, related to the total weight of the semi-finished product,
and
(ii) one or more unpleasant tasting substances of the group consisting of mixtures of amino acids comprising or consisting of each related to the weight, 40 to 50 parts L-leucine, 20 to 30 parts L-isoleucine and 20 to 40 parts L-valine or 10 to 30 parts L-isoleucine, 25 to 45 parts L-leucine, 5 to 15 parts L-phenylalanine and 5 to15 parts L-tryptophan, and peptides with the sequences in an amount of
(A) 0.001 to 10 wt.-%, preferably at least 0.005 wt.-%, at least 0.01 wt.-%, at least 0.05 wt.-%, at least 0.1 wt.-%, at least 0.5 wt.-% or at least 1 wt.-%, related to the total weight of the composition (finished product), or, respectively,
(B) 0.001 to 95 wt.-%, preferably 0.01 to 80 wt.-%, particularly preferably 0.1 to 50 wt.-%, related to the total weight of the semi-finished product.

6. Composition according to claim 5, additionally comprising one, two or more further substances not according to formula (I) for masking an unpleasant, particularly bitter, taste impression of one or more unpleasant tasting substances.

7. Composition according to claims 5 or 6, wherein the or, respectively, one, two, more or all of the hydroxybenzoic acid amides of formula (I) or, respectively, their salt(s) is/are independently of each other selected from the group consisting of
2,4-Dihydroxy-N-[(4-hydroxy-3-methoxy-phenyl)methyl]benzamide (compound 1),
5,7-Dihydroxy-3-[(4-hydroxy-3-methoxy-phenyl)methyl]-1,3-benzoxazin-2,4-dione (compound 2),
4-Hydroxy-N-[(4-hydroxy-3-methoxy-phenyl)methyl]benzamide (compound 3) and
2-Hydroxy-N-[(4-hydroxy-3-methoxy-phenyl)methyl]benzamide (compound 4)
and their salts.

8. Composition according to one of the claims 5 to 7, wherein for the or, respectively, one, two, more or all salts of one, two or more different hydroxybenzoic acid amides of formula (I), as defined in one of the preceding claims, independently applies that the counter ion(s) of the or, respectively, one, more or all of the salts is/are selected from the group consisting of Na⁺ and K⁺.

9. Composition according to one of the claims 5 to 8, wherein the weight ratio of the total amount of component (i) to the total amount of component (ii) of the composition is in a range of 1 : 100,000 to 1 : 500, preferably in a range of 1 : 10,000 to 1 : 50.

10. Composition according to one of the claims 5 to 9, wherein the amount of component (ii) is sufficient to be perceived as unpleasant, preferably bitter, taste in a comparative composition, not comprising component (i), but otherwise having an identical composition of the ingredients, and/or wherein the amount of component (i) is sufficient to mask the unpleasant, preferably bitter, taste impression of the unpleasant, preferably bitter, tasting substance(s) according to component (ii), compared to the comparative composition not comprising component (i).

11. Method for (a) masking the unpleasant, particularly bitter, taste impression of one, two or more unpleasant, particularly bitter, tasting substances in a composition according to one of the claims 5 to 10 and/or (b) producing a preparation according to one of the claims 5 to 10, with the following step:
Contacting or mixing the components (i) and (ii) as well as optionally further components
(i) one, two or more different hydroxybenzoic acid amides of formula (I), as defined in one of the claims 1 to 3, or one, two or more different salts of one, two or more different hydroxybenzoic acid amides of formula (I), as defined in one of the claims 1 to 3, or a mixture of one, two or more different hydroxybenzoic acid amides of formula (I), as defined in one of the claims 1 to 3, with one, two or more different salts of one, two or more different hydroxybenzoic acid amides of formula (I), as defined in one of the claims 1 to 3,
in an amount of
(A) 0.0001 to 0.5 wt.-%, preferably 0.001 to 0.1 wt.-%, related to the total weight of the composition (finished product), or, respectively,
(B) 0.0001 to 95 wt.-%, preferably 0.001 to 80 wt.-%, particularly preferably 0.01 to 50 wt.-%, related to the total weight of the semi-finished product,
(ii) one or more unpleasant tasting substances of the group consisting of mixtures of amino acids comprising or consisting of each related to the weight, 40 to 50 parts L-leucine, 20 to 30 parts L-isoleucine and 20 to 40 parts L-valine or 10 to 30 parts L-isoleucine, 25 to 45 parts L-leucine, 5 to 15 parts L-phenylalanine and 5 to15 parts L-tryptophan, and peptides with the sequences in an amount of
(A) 0.001 to 10 wt.-%, preferably at least 0.005 wt.-%, at least 0.01 wt.-%, at least 0.05 wt.-%, at least 0.1 wt.-%, at least 0.5 wt.-% or at least 1 wt.-%, related to the total weight of the composition (finished product), or, respectively,
(B) 0.001 to 95 wt.-%, preferably 0.01 to 80 wt.-%, particularly preferably 0.1 to 50 wt.-%, related to the total weight of the semi-finished product.

12. Method according to claim 11, wherein the, one, two, more or all of the hydroxybenzoic acid amide(s) of formula (I) or, respectively, their salt(s) is/are independently selected from the group consisting of
2,4-Dihydroxy-N-[(4-hydroxy-3-methoxy-phenyl)methyl]benzamide (compound 1),
5,7-Dihydroxy-3-[(4-hydroxy-3-methoxy-phenyl)methyl]-1,3-benzoxazin-2,4-dione (compound 2),
4-Hydroxy-N-[(4-hydroxy-3-methoxy-phenyl)methyl]benzamide (compound 3) and
2-Hydroxy-N-[(4-hydroxy-3-methoxy-phenyl)methyl]benzamide (compound 4)
and their salts.

13. Method according to one of the claims 11 or 12, wherein it applies for the, one, two, more or all salts of one, two or more different hydroxybenzoic acid amides of formula (I), as defined in one of the preceding claims, that the counter ion(s) of the, one, more or all of the salt(s) is/are selected from the group consisting of Na⁺ and K⁺.

## Revendications

1. Utilisation d'un, de deux ou de plusieurs amides d'acide hydroxybenzoïque différents de formule (I) 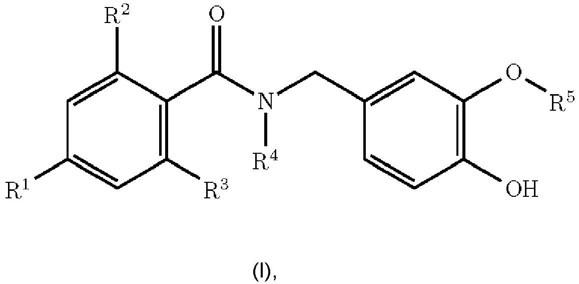 dans laquelle
R¹ et R² signifient, indépendamment l'un de l'autre, l'hydrogène, hydroxy ou méthoxy, à condition qu'au moins l'un des radicaux R¹ ou R² signifie hydroxy,
et
soit
R³ signifie l'hydrogène ou hydroxy
et
R⁴ signifie l'hydrogène
soit
R³ et R⁴ constituent ensemble un groupe -O-C(=O)- et forment ainsi un cycle sous la forme d'un carbamate cyclique,
et
R⁵ signifie l'hydrogène ou le méthyle,
ou
d'un, de deux ou de plusieurs sels différents d'un, de deux ou de plusieurs amides d'acide hydroxybenzoïque différents de formule (I) tels que définis ci-dessus
ou
d'un mélange d'un, de deux ou de plusieurs amides d'acide hydroxybenzoïque différents de formule (I) tels que définis ci-dessus, avec un, deux ou plusieurs sels différents d'un, de deux ou de plusieurs amides d'acide hydroxybenzoïque différents de formule (I) tels que définis ci-dessus
pour masquer une impression de goût désagréable, de préférence un goût ou un arrière-goût amer, astringent, collant, poussiéreux, sec, farineux, rance et/ou métallique, d'une ou de plusieurs substances ayant un goût désagréable, la ou bien une, plusieurs ou la totalité des substances désagréables étant choisie ou bien choisies dans le groupe constitué par des mélanges d'acides aminés contenant ou se composant, respectivement par rapport au poids, de 40 à 50 parties de L-leucine, de 20 à 30 parties de L-isoleucine et de 20 à 40 parties de L-valine, ou de 10 à 30 parties de L-isoleucine, de 25 à 45 parties de L-leucine, de 5 à 15 parties de L-phénylalanine et de 5 à 15 parties de L-tryptophane, et des peptides avec les séquences GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VVV, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF ou YGLF.

2. Utilisation selon la revendication 1, dans laquelle ledit ou bien un, deux, plusieurs ou l'ensemble des amides d'acide hydroxybenzoïque de formule (I) ou bien leur(s) sel(s) est ou bien sont choisi(s) chacun indépendamment les uns des autres dans le groupe constitué par
le 2,4-dihydroxy-N-[(4-hydroxy-3-méthoxy-phényl)méthyl]benzamide (composé 1),
le 5,7-dihydroxy-3-[(4-hydroxy-3-méthoxy-phényl)méthyl]-1,3-benzoxazin-2,4-dione (composé 2),
le 4-hydroxy-N-[(4-hydroxy-3-méthoxy-phényl)méthyl]benzamide (composé 3), et
le 2-hydroxy-N-[(4-hydroxy-3-méthoxy-phényl)méthyl]benzamide (composé 4)
et leurs sels.

3. Utilisation selon l'une quelconque des revendications précédentes, en combinaison avec une, deux ou plusieurs autres substances pour masquer une impression gustative désagréable, en particulier amère, d'une ou de plusieurs substances à goût désagréable.

4. Utilisation selon l'une quelconque des revendications précédentes dans une préparation choisie dans le groupe constitué par
- des préparations (produits finis) orales pharmaceutiques ou cosmétiques, servant à la nutrition ou à l'hygiène buccale et servant à l'application au niveau de la tête, et
- des produits semi-finis, en particulier ceux destinés à la fabrication d'une préparation (produits finis) orale pharmaceutique ou cosmétique, servant à la nutrition ou à l'hygiène buccale et servant à l'application au niveau de la tête, de préférence sous la forme d'une composition odoriférante, d'arôme ou d'agent de sapidité ou d'un mélange d'assaisonnement.

5. Préparation choisie dans le groupe constitué par
(A) des préparations (produits finis) orales pharmaceutiques ou cosmétiques, servant à la nutrition, au plaisir ou à l'hygiène buccale et servant à l'application au niveau de la tête, et
(B) des produits semi-finis, en particulier ceux destinés à la fabrication d'une préparation (produits finis) orale pharmaceutique ou cosmétique, servant à la nutrition, au plaisir ou à l'hygiène buccale et servant à l'application au niveau de la tête, de préférence sous la forme d'une composition odoriférante, d'arôme ou d'agent de sapidité ou d'un mélange d'assaisonnement,
contenant
(i) un, deux ou plusieurs amides d'acide hydroxybenzoïque différents de formule (I) tels que définis dans l'une quelconque des revendications 1 à 3, ou un, deux ou plusieurs sels différents d'un, de deux ou de plusieurs amides d'acide hydroxybenzoïque différents de formule (I), tels que définis dans l'une quelconque des revendications 1 à 3, ou un mélange d'un, de deux ou de plusieurs amides d'acide hydroxybenzoïque différents de formule (I) tels que définis dans l'une quelconque des revendications 1 à 3, avec un, deux ou plusieurs sels différents d'un, de deux ou de plusieurs amides d'acide hydroxybenzoïque différents de formule (I) tels que définis dans l'une quelconque des revendications 1 à 3,
en une quantité de
(A) comprise entre 0,0001 et 0,5 % en poids, de préférence entre 0,001 et 0,1 % en poids, par rapport au poids total de la préparation (produit fini), ou bien
(B) comprise entre 0,0001 et 95 % en poids, de préférence entre 0,001 et 80 % en poids, de manière particulièrement préférée entre 0,01 et 50 % en poids, par rapport au poids total du produit semi-fini,
et
(ii) une ou plusieurs substances à goût désagréable du groupe constitue par des mélanges d'acides aminés contenant ou se composant, respectivement par rapport au poids, de 40 à 50 parties de L-leucine, de 20 à 30 parties de L-isoleucine et de 20 à 40 parties de L-valine, ou de 10 à 30 parties de L-isoleucine, de 25 à 45 parties de L-leucine, de 5 à 15 parties de L-phénylalanine et de 5 à 15 parties de L-tryptophane, et des peptides avec les séquences GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VVV, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF ou YGLF,
en une quantité de
(A) comprise entre 0,001 et 10 % en poids, de préférence au moins 0,005 % en poids, au moins 0,01 % en poids, au moins 0,05 % en poids, au moins 0,1 % en poids, au moins 0,5 % en poids ou au moins 1 % en poids, par rapport au poids total de la préparation (produit fini), ou bien
(B) comprise entre 0,001 et 95 % en poids, de préférence entre 0,01 et 80 % en poids, de manière particulièrement préférée entre 0,1 et 50 % en poids, par rapport au poids total du produit semi-fini.

6. Préparation selon la revendication 5, comprenant en outre une, deux ou plusieurs autres substances ne correspondant pas à la formule (I), pour masquer une impression gustative désagréable, en particulier amère, d'une ou de plusieurs substances à goût désagréable.

7. Préparation selon la revendication 5 ou 6, dans laquelle le ou bien un, deux, plusieurs ou l'ensemble des amides d'acide hydroxybenzoïque de formule (I) ou bien leur(s) sel(s) est ou bien sont choisi(s) chacun indépendamment les uns des autres dans le groupe constitué par
le 2,4-dihydroxy-N-[(4-hydroxy-3-méthoxy-phényl)méthyl]benzamide (composé 1),
le 5,7-dihydroxy-3-[(4-hydroxy-3-méthoxy-phényl)méthyl]-1,3-benzoxazin-2,4-dione (composé 2),
le 4-hydroxy-N-[(4-hydroxy-3-méthoxy-phényl)méthyl]benzamide (composé 3) et
le 2-hydroxy-N-[(4-hydroxy-3-méthoxy-phényl)méthyl]benzamide (composé 4)
et leurs sels.

8. Préparation selon l'une quelconque des revendications 5 à 7, dans laquelle pour le ou un, deux, plusieurs ou l'ensemble des sels d'un, de deux ou de plusieurs amides d'acide hydroxybenzoïque différents de formule (I) tels que définis dans l'une quelconque des revendications précédentes vaut, respectivement indépendamment les uns des autres, que le ou les contre-cation(s) du ou bien d'un, de plusieurs ou de l'ensemble des sels est ou bien sont choisi(s) dans le groupe constitué par Na⁺ und K⁺.

9. Préparation selon l'une quelconque des revendications 5 à 8, dans laquelle le rapport pondéral de la quantité totale de composant (i) à la quantité totale de composant (ii) dans la préparation se situe dans la gamme allant de 1 : 100 000 à 1 : 500, de préférence dans la gamme allant de 1 : 10.000 à 1 : 50.

10. Préparation selon l'une quelconque des revendications 5 à 9, dans laquelle la quantité de composant (ii) est suffisante pour être perçue comme un goût désagréable, de préférence amer, dans une préparation comparative qui ne contient pas de composant (i) mais est par ailleurs composée de façon identique, et/ou dans laquelle la quantité de composant (i) est suffisante pour masquer l'impression gustative désagréable, de préférence amère, de la ou bien des substance(s) à goût désagréable, de préférence amer, selon le composant (ii), en comparaison de la préparation comparative qui ne contient pas de composant (i).

11. Procédé pour (a) masquer l'impression gustative désagréable, en particulier amère, d'une, de deux ou de plusieurs substances à goût désagréable, en particulier amer, dans une préparation selon l'une quelconque des revendications 5 à 10, et/ou pour (b) produire une préparation selon l'une quelconque des revendications 5 à 10, comprenant l'étape suivante consistant à:
mettre en contact ou mélanger les composants (i) et (ii) ainsi que, en option, d'autres composants
(i) un, deux ou plusieurs amides d'acide hydroxybenzoïque différents de formule (I) tels que définis dans l'une quelconque des revendications 1 à 3, ou un, deux ou plusieurs sels différents d'un, de deux ou de plusieurs amides d'acide hydroxybenzoïque différents de formule (I), tels que définis dans l'une quelconque des revendications 1 à 3, ou un mélange d'un, de deux ou de plusieurs amides d'acide hydroxybenzoïque différents de formule (I) tel que définie dans l'une quelconque des revendications 1 à 3, avec un, deux ou plusieurs sels différents d'un, de deux ou de plusieurs amides d'acide hydroxybenzoïque différents de formule (I) tels que définis dans l'une quelconque des revendications 1 à 3,
en une quantité de
(A) comprise entre 0,0001 et 0,5 % en poids, de préférence entre 0,001 et 0,1 % en poids, par rapport au poids total de la préparation (produit fini), ou bien
(B) comprise entre 0,0001 à 95 % en poids, de préférence entre 0,001 à 80 % en poids, de manière particulièrement préférée entre 0,01 et 50 % en poids, par rapport au poids total du produit semi-fini,
(ii) une ou plusieurs substances à goût désagréable du groupe constitué par des mélanges d'acides aminés contenant ou se composant, respectivement par rapport au poids, de 40 à 50 parties de L-leucine, de 20 à 30 parties de L-isoleucine et de 20 à 40 parties de L-valine, ou de 10 à 30 parties de L-isoleucine, de 25 à 45 parties de L-leucine, de 5 à 15 parties de L-phénylalanine et de 5 à 15 parties de L-tryptophane et des peptides avec les séquences GKHQQEEENEGG, NFNNQLDQTPR, AGNPDIEHPE, NALEPDHRVE, GNPDIEHP, IYPGCPST, KLHENIAR, LAGNQEQE, ALEPDHR, EQGGEQG, EQPQQNE, IGTLAGA, NAMFVPH, GMIYPG, HNIGQT, IYPGCP, NALKPD, FIQGV, NALPE, NNEDT, SAEFG, SIIDT, YEGNS, LLLL, NLQG, SDNF, EGG, GAL, GGL, KPF, LLL, PPG, AD, EG, EY, GE, GF, GI, IA, IP, IS, KP, LK, PR, PY, RG, RP, RR, VF, VI, VV, VY, VEELKPTPEGDLEIL, LKP, LVL, DL, ID, LD, LE, LV, WE, QLFGPNVNPWHNP, QLFNPSTNPWHSP, GGRGPPFIVGG, QLFNPSTNPWH, GGRGPPFIV, QLFNPSTNP, RGPPFIVGG, RGPPGGGFF, GGRPFFGG, QLFNPS, GFG, GLL, GVV, LQL, RPG, EF, FG, FV, GL, GR, GV, IE, II, IL, IQ, LF, LI, RF, VA, WF, YF, YG, AQTQSLVYPFPGPIPNSLPQNIPPLTQ, YQQPVLGPVRGPFPIIV, PVLGPVRGPFPIIV, SLVYPFPGPIHNS, VPLGTQYTDAPSF, FFVAPFPEVFGK, FFVAPFPQVFGK, LVYPFPGPIHN, PVRGPFPIIV, VYPFPGPIPN, VYPFPPIGNH, YLGYLEQLLR, YPFPGPIHNS, YPFPGPIPNS, PFPGPIPNS, YPFPGPHIN, YPFPGPIPN, FALPQYLK, GPVRGPFP, LGYLEQLL, RGPFPIIV, RGPGPIIV, APFPEVF, AYFYPEL, FYPELFR, PFPGPIP, RGPFPIV, YPFPGPI, PVLGPV, VRGPFP, FYPELF, GPFPIV, PFPGPI, PFPIIV, DIKQM, EIVPN, NENLL, PGPIP, EVLN, LPQE, VYPF, APK, EEN, FLL, FPK, FPP, LRF, LRL, PFP, PGP, PPF, RGP, VVV, VYP, YPF, AF, AL, AV, DA, EI, EL, FF, FI, FL, FP, FY, GP, GY, IF, IG, IK, IN, IT, IV, KF, LG, LW, LY, MI, PF, PI, PK, PL, PP, RL, SL, VD, VE, VL, YP, YY, GPFPVI, FALPEYLK, VG, RGPPFIV, GRP, FFF, WW, WWW, YPFP, FPF, IPAVF, LLF ou YGLF,
en une quantité de
(A) comprise entre 0,001 et 10 % en poids, de préférence au moins 0,005 % en poids, au moins 0,01 % en poids, au moins 0,05 % en poids, au moins 0,1 % en poids, au moins 0,5 % en poids ou au moins 1 % en poids, par rapport au poids total de la préparation (produit fini), ou bien
(B) comprise entre 0,001 et 95 % en poids, de préférence entre 0,01 et 80 % en poids, de manière particulièrement préférée entre 0,1 et 50 % en poids, par rapport au poids total du produit semi-fini.

12. Procédé selon la revendication 11, dans lequel le ou bien un, deux, plusieurs ou l'ensemble des amides d'acide hydroxybenzoïque de formule (I) ou bien leur(s) sel(s) est ou bien sont choisi(s) chacun indépendamment les uns des autres dans le groupe constitué par
le 2,4-dihydroxy-N-[(4-hydroxy-3-méthoxy-phényl)méthyl]benzamide (composé 1)
le 5,7-dihydroxy-3-[(4-hydroxy-3-méthoxy-phényl)méthyl]-1,3-benzoxazin-2,4-dione (composé 2),
le 4-hydroxy-N-[(4-hydroxy-3-méthoxy-phényl)méthyl]benzamide (composé 3) et
le 2-hydroxy-N-[(4-hydroxy-3-méthoxy-phényl)méthyl]benzamide (composé 4)
et leurs sels.

13. Procédé selon la revendication 11 ou 12, dans lequel pour le ou un, deux, plusieurs ou l'ensemble des sels d'un, de deux ou de plusieurs amides d'acide hydroxybenzoïque différents de formule (I) tels que définis dans l'une quelconque des revendications précédentes vaut, respectivement indépendamment les uns des autres, que le ou les contrecation(s) du ou bien d'un, de plusieurs ou de l'ensemble des sels est ou bien sont choisi(s) dans le groupe constitué par Na⁺ und K⁺.
